# EUROPEAN PATENT APPLICATION

(11) **EP 1 413 273 A1**
(43) Date of publication of application: **28.04.2004**
(21) Application number: 02771753.7
(22) Date of filing: 21.05.2002
(51) Int. Cl.: A61F 13/15, B65D 81/26, B65D 85/16

(54) **INDIVIDUAL PACKAGING BODY AND OUTER VESSEL THEREFOR**

(30) Priority: 22.05.2001 JP 2001152403; 17.12.2001 JP 2001383059
(71) Applicant: UNI-CHARM CORPORATION, Kawanoe-shi Ehime 799-0111 (JP)
(72) Inventor: MIZUTANI, Satoshi Technical Center, Uni-Charm Corp, Mitoyo-gun, Kagawa 769-1602 (JP); YAMAKI, Koichi Technical Center, Uni-Charm Corp., Mitoyo-gun, Kagawa 769-1602 (JP); NODA, Yuki c/o Technical Center, Uni-Charm Corp., Mitoyo-gun, Kagawa 769-1602 (JP)
(74) Representative: Purdy, Hugh Barry
(86) International application number: PCT/JP2002/004893
(87) International publication number: WO 2002/094157

(57) **Abstract**

The present invention relates to an individual wrapping body for wrapping an interlabial pad in which the number of live microorganism is suppressed and an exterior container for enclosing two or more of the individual wrapping bodies. An object is to provide an individual wrapping body capable of preventing microorganism from entering to female genital from outside and maintaining the state of equilibrium of indigenous microorganism. The individual wrapping body comprises an interlabial pad and an individual wrapping container for covering and enclosing the whole portion of the interlabial pad. A processing for suppressing the number of live microorganism is applied to the interlabial pad and, by the processing, the number is suppressed to be 100 or less even a period of 6 months after the manufacture. Also, provided is a package for surrounding two or more of the individual wrapping bodies.

## Description

### Background of the Invention

### Technical Field

The present invention relates to an individual wrapping body for wrapping an interlabial pad in which the number of live microorganism is suppressed and an exterior container for enclosing two or more of the individual wrapping body.

### Background Art

Conventionally, a sanitary napkin and a tampon are used generally as sanitary products for female. Here, there have been great efforts made to prevent the leak of menstrual blood from gap caused by poor adhesion near the ostium vaginae as for the sanitary napkin. Moreover, as for the tampon, there have been great efforts made for relieving the foreign feeling and the discomfort when wearing the sanitary products and intervaginal wearing trouble due to the nature of those products.

Under such situation, sanitary products of the interlabial pad have attraced people as a sanitary product positioned between the sanitary napkin and the tampon in recent years.

The interlabial pad is fixed by inserting it between the labia, having characteristics that it is difficult to cause the leak of menstrual blood because of higher adhesion to the body than that of the sanitary napkin and psychological resistance thereof on wearing is lower than that of the tampon which is inserted into the vagina.

An example of sanitary products requiring a disinfected sanitary state is a tampon which is used by being inserted inside the vagina. Tampons are individually wrapped and then sterilized thereby preventing microorganism from entering inside the vagina from outside through the product. Therefore, the state of equilibrium of indigenous microorganism can be maintained even when wearing a tampon, thereby keeping the sanitary state.

Tampons can be broadly divided into two groups such as an applicator tampon, which is inserted inside the vagina using an applicator, and a finger-type tampon which is inserted inside the vagina using fingers.

As for the former, there is no chance that the tampon comes to be in contact with fingers of a wearer or the like at least until the insert, so that the sanitary state after the sterilization can be maintained. However, as for the latter, it is common that a wearer hold the tampon for insert by the thumb and middle finger and then insert it by the forefinger to which an enclosed finger cap is applied. Therefore, although entering of microorganism inside the vagina through the forefinger can be prevented, the tip of thumb and middle finger holding the tampon are to be in contact with the tampon.

An interlabial pad is capable of directly absorb menstrual blood from the labia near the ostium vaginae, unlike a sanitary napkin used by being fixed to an underwear or the like, and the pad is used by being continuously pinched by the labia near the ostium vaginae upon wearing. The labia exhibits no bacteriostatic action, like the self-cleaning action of the vagina, against the microorganism entering from outside. Therefore, in the interlabial pad, a more sanitary environment is required than in the sanitary napkin.

However, in interlabial pads of the related art, there has been no control over the number of live microorganism which thrive in the interlabial pad before being used. Also, a tampon is an example of a wearing system which can keep the sanitary state of the product until when it is worn. However, as described earlier, in the case of a finger tampon, the microorganism in the tip of the finger can enter inside the vagina. Therefore, in regards to preventing the microorganism from entering from the outside and maintaining the state of equilibrium of the indigenous bacteria, it is imperfect as a system to keep the sanitary state.

Usually, although there is a personal difference in the type of bacteria, for example, near the skin, there are staphylococcus epidermidis, non-hemolytic streptococcus, mycetes, or, even though very little, staphylococcus aureus, hemolytic streptococcus and the like thriving as the skin indigenous microorganism nest (indigenous microorganism), keeping a specific biological equilibrium state with each other, and also between the microorganism and the living body.

Naturally, the same is true in the area near the labia, however, there may be a case where it becomes hard to keep the state of equilibrium of the indigenous bacteria near the labia if bacteria enters near the labia from outside by an absorbent article and the like.

Accordingly, although it depends on each individual, the state of equilibrium of the indigenous microorganism near the labia is disturbed so that microbes may easily propagate, which may cause to contract a disease in external genitalia and internal genitalia including the labia.

### Summary of the Invention

The present invention has been designed to overcome the foregoing problems. An object of the present invention is to provide an individual wrapping body for wrapping an interlabial pad, which can keep the sanitary state of female labia, and an exterior container for enclosing the individual wrapping body.

The present invention provides an interlabial pad in which the number of live microorganism which thrive before being used is suppressed and its individual wrapping body and wrapping container.

Also, the present invention can provide an interlabial pad having a wearing-handling property in which there is no need for the fingers or the like of a user to be in contact with the face of the interlabial pad facing to the skin. Therefore, in the interlabial pad, the state of equilibrium of the indigenous microorganism near the labia can be maintained.

More specifically, the present invention provides the followings.

(1) An individual wrapping body comprising: an interlabial pad with a size capable of being smoothly inserted between female labia; and an individual wrapping container for covering and enclosing the whole portion of said interlabial pad, wherein a processing for suppressing the number of live microorganism is applied to said interlabial pad during manufacturing and / or after manufacturing, and the number of live microorganism is suppressed to equal to 100 or less even after a period of six months from the manufacture.

In the interlabial pad, the number of live microorganism is suppressed to be 100 or less even 6 months after the manufacture. Therefore, by wearing the interlabial pad, the state of equilibrium of the indigenous microorganism near the labia is not to be disturbed, thereby further decreasing the risk of contracting diseases in the external genitalia and internal genitalia.

Examples of the measure for suppressing the number of live microorganism are disinfection processing, manufacturing under aseptic condition, and using antimicrobial agent.

Examples of the disinfention processing are gassing, an irradiation method, and a radiation method. Gassing is a method in which microbes are killed using ethylene oxide, formaldehyde, hydrogen peroxide or other gases. Examples of irradiation method also include ultraviolet method, high frequency method and electron ray method. The ultraviolet method is a method for perishing the microbes by irradiating ultraviolet rays. The high frequency method is a method for perishing the microbes through the heat generated by directly applying the high frequency. The electron ray method is a method for perishing the microbes by irradiating the electron rays. The radiation method is a method in which microbes are perished by irradiating radiation such as gamma radiation from the source including radioactive isotope element.

To manufacture under an aseptic condition refer to the manufacture in which all the manufacturing steps are performed under an aseptic condition using materials which are disinfected or sterilized beforehand or using materials which are proved to be aseptic.

Usage of an antimicrobial agent is to use the antimicrobial agent on either the interlabial pad or the individual wrapping container, or on both. In order to suppress the number of live microorganism in the interlabial pad itself, it is preferable that the interlabial pad itself contain the antimicrobial agent. The method of containing in the interlabial pad is selected from the methods well known to the those skilled in the art with the consideration of cost and safety of the users and the like. As the antimicrobial agent, there are a chemosynthetic type (organic type, inorganic type) antimicrobial agent and an antimicrobial agent originated from natural substances. In order to provide a sense of safety to users, that is, to the wearers of the interlabial pad, the antimicrobial agent originated from the natural substances is preferable. However, a chemosynthetic type antimicrobial agent may be also used if proved that it is low stimulant and high safety.

The disinfection processing, manufacture under the aseptic condition, and usage of the antimicrobial agent may all be performed in combination.

(2) An individual wrapping body according to (1), wherein said processing is a disinfection processing.

The disinfection processing is a processing to disinfect microbes, and sterilization is a processing performed to completely perishing the whole microbes. Hence, in the present invention, sterilization includes the disinfection processing.

(3) An individual wrapping body according to (2), wherein, by said disinfection processing, said interlabial pad is sterilized immediately after manufacturing.

As described, sterilization is a processing performed to completely perish the whole microbes. It is ideal to check whether or not the interlabial pad immediately after the manufacture is surely sterilized by inspecting all the products after the manufacture. However, it is practically impossible. Therefore, in the present invention, SAL; Sterility Assurance Level is employed. SAL shows the degree of an aseptic state in the products achieved after the sterilization step, and is expressed by the anticipated thriving probability of microbes in the sterilized products per unit. SAL is usually expressed by 10⁻ⁿ. In the present invention, n is not less than 3, and preferably n is not less than 6. When n = 3, it allows the live bacteria thriving in 1 out of 1000 interlabial pads after the manufacture. In other words, the number of live microorganism per product is 1 / 1000. If SAL is kept within such range, there is little likelihood that the bacteria propagate while the interlabial pad is being preserved.

(4) An individual wrapping body according to any one of (1) to (3), wherein said interlabial pad comprises: a permeable surface side sheet facing a body side; a back side sheet facing a garment side, the back side sheet being bonded to said surface side sheet; and an absorber included between said surface side sheet and said back side sheet.

The interlabial pad may be in a flat shape or may be rolled so that a finger can be inserted and the back side sheet becomes the inner side.

(5) An individual wrapping body according to any one of (1) to (4), further comprises a mini sheet piece on the garment side surface of said back side sheet, said mini sheet piece having one or more bonded areas in each side area in the longitudinal direction of said back side sheet; and an unbonded area in the lateral direction of said back side sheet; said unbonded area forming an opening for inserting a finger for inserting a finger between said mini sheet piece and said back side sheet.

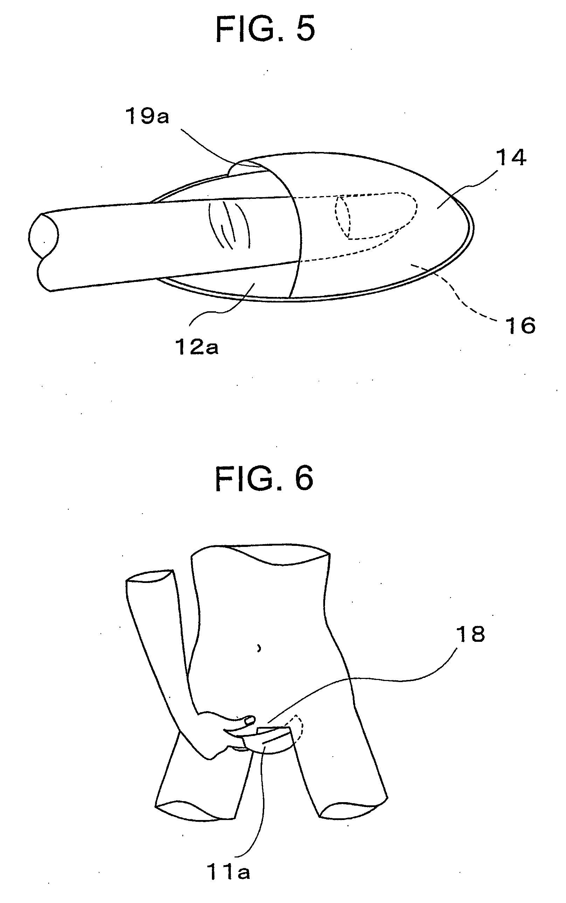

In the interlabial pad according to the embodiment, a mini sheet piece is attached on,the garment side face. As for the mini sheet piece, in the lateral direction of the back side sheet, at least one of both sleeve ends of a mini sheet piece is unbonded with the face of the back side sheet. Thus, there is a sleeve opening formed between the one of the sleeve portion of the mini sheet piece and the back side sheet which are unbonded. The sleeve opening serves as an opening for inserting a finger (see Fig. 5) to which a finger can be inserted.

In the longitudinal direction of the back side sheet, the mini sheet piece is bonded only on the right and left hand sides of the back side sheet, and the inside is unbonded (not pasted). Therefore, the mini sheet piece is attached over the area from one side to the other side of the back side sheet. In the area from one side to the other side, a space is formed (finger insert space) to which a finger can be inserted for holding.

In the lateral direction of the back side sheet, if both of the sleeve ends of the mini sheet piece are unbonded with the back side sheet, the space for inserting finger becomes a through hole (a tunnel). When one of the sleeve ends of the mini sheet piece is bonded, the space for inserting finger becomes a non-through cave.

If the mini sheet piece comprises a plurality of pieces, the number of the bonding area on the right and left hand sides increases on the back side sheet in the longitudinal direction according to the number of the pieces. For example, if there are two mini sheet pieces, two bonding areas are provided on each side.

In the present invention, "side area" of the back side sheet in the longitudinal direction include not only the area corresponding to the peripheral edge of the interlabial pad but also the neighborhood of the peripheral edge where the mini sheet piece can be bonded.

As described, according to the embodiment, the mini sheet piece is attached to form the opening for inserting a finger and the continued gap for finger insert. Therefore, by inserting a finger to the opening for inserting a finger, the interlabial pad can be temporarily fixed to the tip of a finger. In this case, the opening for inserting a finger is formed to be the aperture with the finger breadth of the wearer so that the flat-shape finger tip is to be inserted to naturally be in contact with the face of the sheet without facing the different direction from the face of the sheet. In other word, in the interlabial pad according to the present invention, the opening for inserting a finger has a widespread shape towards the direction of the sheet surface as in the finger tip shape of the wearer. Therefore, the finger insert direction of the wearer is specified thereby leading the wearer to detect the wearing point by the ball of finger. Thus, even when wearing the pad between the labia, which is a place hard to view, the interlabial pad can be inserted to an appropriate position while accurately detecting a precise wearing position. In addition, when wearing the interlabial pad inside the labia, the interlabial pad can be inserted by detecting between the labia using the inserted finger so that there is little risk that a finger accidentally touches the surface side sheet or taking too much time for wearing. For the reasons described above, there is little risk that microorganism attaches the surface side sheet and, in addition, microorganism enter the labia.

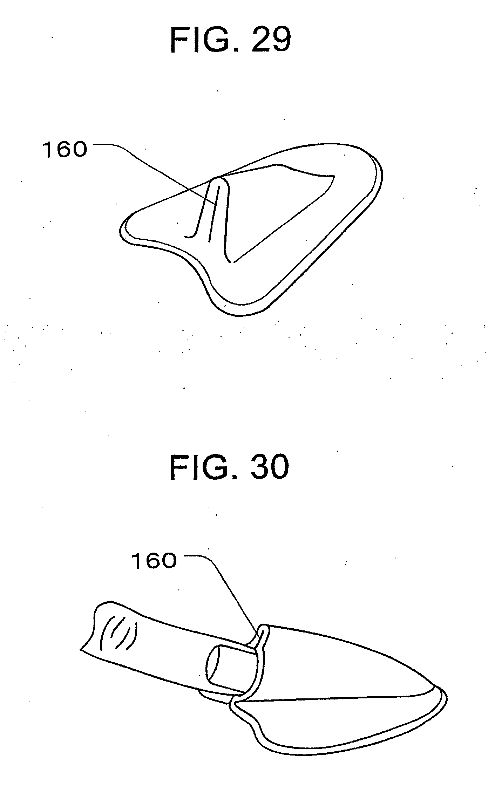

Incidentally, JP-A-Hei 6-506368 discloses a pad for preventing incontinence of urine in which a bag shaped hole for inserting a finger is provided on the opposite side surface to the body side. However, it mentions, regarding the above described opening for inserting a finger, that "it is crushed in a normal state and spread open after inserting a finger". This indicates that the hole is closed in a normal state (see Fig. 29). Therefore, a finger cannot be inserted without making the finger face in the direction with a right angle to the pad for preventing incontinence of urine (see Fig. 30). The ball of finger cannot face the interlabial pad side unless the finger is turned around after inserted. However, when performing this action, the pad for preventing incontinence of urine is turned around together with the finger unless the pad for preventing incontinence of urine is supported by the other hand. Hence, the finger cannot be set in the desired position.

As described, unlike the present invention, the finger insert direction of the opening for inserting a finger provided in the above-described pad for preventing incontinence of urine is specified to form the right angle against the sheet surface. Therefore, the finger is not inserted with the ball of finger facing the sheet surface in the first place. Thus, the effect of simple and rapid wearing cannot be sufficiently achieved.

In the present invention, the position of the opening for inserting a finger can be changed appropriately. However, it is desirable to provide the positioning such that the size of the finger insert gap continued from the opening for inserting a finger becomes sufficient for inserting a finger to fix and support the interlabial pad (the wearer can feel the finger as one body with the interlabial pad).

In this Specification, the "finger breadth" specifically means "the width of a finger in the direction of the finger spread" but not the thickness of the finger. The "finger breadth-opening" is an opening having a size sufficient for inserting a finger.

Also, the finger breadth-opening "is directly provided" in the face direction of the interlabial pad means that the interlabial pad itself is formed to be in a suitable shape primarily in the case where a finger is naturally inserted to the interlabial pad for wearing the interlabial pad (in the present invention, when inserting a finger with the ball of finger facing the sheet surface of the back side. sheet). Therefore, it can eliminate such case that, as in the above-described related art, the wearer can only obtain the opening of the finger breadth in the surface direction by inserting the finger and then turning around the finger, in which the finger breadth-opening is secondarily formed in the surface direction of the back side sheet.

(6) An individual wrapping body according to (5), wherein said interlabial pad is folded and enclosed such that said opening for inserting a finger opens.

In a sheet shaped sanitary product such as a napkin, it is common that the content is folded and wrapped for minimizing the size of the wrapping body. However, in the present invention, in addition to minimizing the size, there is an effect obtained in which the opening for inserting a finger is to be naturally opened by simply opening the folded interlabial pad. The opening for inserting a finger is naturally opened so that it becomes easy to insert the finger by recognizing the opening for inserting a finger. Thus, there is little risk that the finger accidentally touches the surface side sheet or the wearer takes too much time for wearing. As a result, there is little risk that the microorganism attach to the surface side sheet, and that the microorganism enters the labia.

(7) An individual wrapping body according to (5) or (6), wherein an interlabial pad is positioned such that said opening for inserting a finger is positioned to face the opening of said individual wrapping body.

The interlabial pad is positioned inside an individual wrapping body such that a finger can be inserted to the opening for inserting a finger upon opening the individual wrapping body. Hence, the interlabial pad is easily taken out from the individual wrapping body. The interlabial pad may be, for example, folded in two or may not be folded. When folded, it may be folded in the longitudinal direction or in the lateral direction. When the wearer opens the wrapping body and takes out the interlabial pad from the wrapping body, there is no way that the finger touches the surface side sheet. As a result, there is little risk that the microorganism attaches to the surface side sheet and that the microorganism enter the labia.

(8) An exterior container, comprising a package for packaging two or more of said individual wrapping bodies according to any one of claims (1) to (7), wherein said package comprises a main body of a container and a lid capable of opening and closing for covering an opening part of said main body of the container.

By using a package, a plurality of individual wrapping bodies can be sold as a unit and preservation of the individual wrapping body becomes easy. The package may have an airtight characteristic sealing the interlabial pad or may be air-permeable.

After opening the opening part of the main body of the container, by covering the opening part with a lid capable of opening and closing, dropping and mixing of contamination such as floating dust and floating microbes (microbes in the air) can be decreased to an extreme extent. Thereby, the pad can be preserved under a sanitary environment.

(9) An exterior container according to (8), wherein said package includes an over - wrapping for covering at least said main body of the container.

Even if there is a gap between the main body of the container and the lid, the gap can be covered by over-wrapping. By using the over-wrapping, entering of the microbes in the air to the interlabial pad becomes more difficult. It is preferable that the over-wrapping covers most of the part or the whole portion of the main body of the container and the lid.

(10) An exterior container according to (8) or (9), wherein said main body of the container of said package is bonded with at least a part of said lid.

Opening and closing of the lid become easy so that the individual wrapping body inside the main body of the container can be easily taken out. The lid is not completely separated from the main body so that not only opening and closing can perform rapidly, but also no chances that the lid is lost and that the lid cannot be found immediately. As a result, the opening of the main body of the container can be surely and easily covered. Thereby, decreasing the dropping and mixing of the contamination such as floating dust and floating microbes (microbes in the air) to an extreme extent and the pad can be preserved under a sanitary environment.

(11) An exterior container according to (8) or (9), wherein: said main body of the container of said package and said lid are continuously formed; and said package is formed by folding said lid towards said main body of the container.

The main body of the container and the lid, for example, are formed by a piece of cardboard. There may be the case where the cardboard is folded and composed to form a package. It can be manufactured at a low cost.

### Brief Description of the Drawings

Fig. 1 is a cross section showing the inner structure of an interlabial pad;
Fig. 2 is a plan view showing the top face (body side) of the interlabial pad;
Fig. 3 is a perspective view showing the reverse surface (garment side) of the interlabial pad;
Fig. 4 is a cross sectional explanatory illustration for describing the attaching position of a mini sheet piece of the interlabial pad;
Fig. 5 is a perspective view showing the state where a finger is inserted to a finger insert pocket provided in the interlabial pad;
Fig. 6 is a perspective view showing the state of wearing the interlabial pad between the labia;
Fig. 7 is a plan view showing the state where the mini sheet piece attached to the interlabial pad has a length of 10 percent or more in the longitudinal direction;
Fig. 8 is a perspective view showing the open state of an individual wrapping body which wraps an interlabial pad with the back side sheet being folded inside;
Fig. 9 is a perspective view showing the open state of the case where the interlabial pad according to the embodiment is wrapped in an individual wrapping body which opens as a set of folded doors;
Fig. 10 is a perspective view showing the open state of an individual wrapping body which wraps an interlabial pad with the surface side sheet being folded inside;
Fig. 11 is a perspective view showing the open state of the case where the interlabial pad is folded and wrapped inside an individual wrapping body which is opened by stripping off the top face;
Fig. 12 is a perspective view showing the open state of the case where the interlabial pad is folded and wrapped inside an individual wrapping body which is opened by stripping off the top face;
Fig. 13 is a perspective view showing the open state of the case where the interlabial pad is left unfolded and wrapped inside an individual wrapping body which is opened by stripping off the top face;
Fig. 14 is a plan view of an individual wrapping body according to the embodiment;
Fig. 15 is a plan view showing an individual wrapping body according to the embodiment;
Figs. 16(A), 16(B) and 16(C) are plan views of an individual wrapping body according to the embodiment, while Fig. 16(A) shows the state before being opened, Fig. 16(B) shows the state after being opened, and Fig. 16(C) shows the explanatory illustration at the time of wearing;
Fig. 17 is a perspective view showing a package according to an embodiment;
Fig. 18 is a perspective view showing a package according to another embodiment;
Fig. 19 is a perspective view showing a package according to still another embodiment;
Figs. 20(A) and 20(B) are perspective views showing a package according to a further embodiment, while Fig. 20(A) shows the state where a lid is closed and Fig. 20(B) shows the state where the lid is slightly open;
Figs. 21(A) and 21(B) are explanatory illustrations of over-wrapping while Fig. 21(A) shows the state before the application of over-wrapping and Fig. 21(B) shows the state after the application of over-wrapping;
Figs. 22(A) and 22(B) are explanatory illustrations of a package with a sealing member while Fig. 22(A) shows the state where the lid is open and Fig. 22(B) shows the state where the lid is closed;
Figs. 23(A), 23(B), and 23(C) are explanatory illustrations of a package according to the embodiment while Fig. 23(A) shows the unfolded view of the package according to the embodiment, Fig. 23(B) shows a perspective view of the package according to the embodiment during the composing step, and Fig. 23(C) is a perspective view showing the state after the package is composed;
Figs. 24(A), 24(B) and 24(C) are explanatory illustrations of the over-wrapping while Fig. 24(A) is a perspective view showing a gap, Fig. 24(B) is a perspective view showing the state before wrapping, and Fig. 24(C) is a perspective view showing the state after wrapping;
Fig. 25 is a perspective view showing the experimental state of separation force measurement of an anti-shift tape;
Fig. 26 is a perspective view showing the experimental state of shearing force measurement of an anti-shift tape;
Figs. 27(A) and 27(B) are explanatory illustrations showing an example of embodiment of the individual wrapping body.while Fig. 27(A) is a plan view and Fig. 27(B) is a cross section along the line X-X;
Fig. 28 is a perspective view showing the state of gas entering inside the package;
Fig. 29 is a perspective view showing the state of an example of a pad for preventing incontinence of urine with an opening for inserting a finger according to the related art; and
Fig. 30 is a perspective view for describing the state of finger insert of in the pad for preventing incontinence of urine with an opening for inserting a finger according to the related art.

### Best Mode of Carrying Out the Invention

Now, the embodiment of the present invention will be described by referring to the drawings.

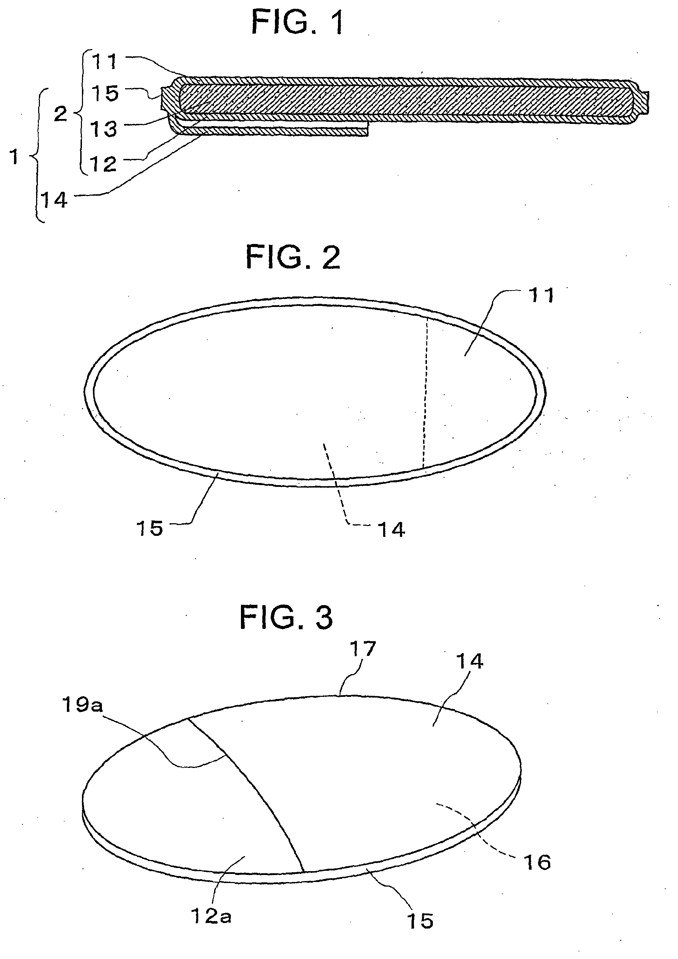

Fig. 1 is an illustration showing the inner structure of an interlabial pad according to the embodiment. Fig. 2 and Fig. 3 are illustrations showing the appearance of the interlabial pad according to the embodiment. Fig. 2 is an illustration showing the body side face of the interlabial pad according to the embodiment and Fig. 3 is an illustration showing the garment side face of the interlabial pad according to the embodiment. Fig. 5 and Fig. 6 are explanatory illustrations showing the state of using the interlabial pad.

### [Basic Structure]

An interlabial pad 1 according to the embodiment, as shown in Fig. 1, comprises a surface side sheet 11 made of a permeable material, a back side sheet 12 and an absorber 13. The surface side sheet 11 and the back side sheet 12 are bonded in a peripheral edge 15 so as to seal the absorber 13 inside thereby forming an absorber layer 2 as one body. The surface side sheet 11 and the back side sheet 12 are bonded by heat embossing and / or hot melt adhesive. The absorber 13 is pasted to each sheet so as to prevent the interlayer separation between the surface side sheet 11 and the back side sheet 12.

Incidentally, the above - described interlayer separation can be prevented by pasting the surface side sheet 11 and the back side sheet 12 in the inner edge except for the peripheral edge 15. Also, under a wet condition, the interlayer separation is likely to occur. In order to prevent this, it is more preferable to use heat embossing. Heat embossing can be used in dots or screen pattern without limit. By bonding them with the embossing surface ratio from 3 to 20 percent, it becomes possible to keep the moist strength without interrupting the permeability.

The interlabial pad can be used, in addition to absorbing menstrual blood, for absorbing secretion (vaginal discharge) other than the blood discharged from the ostium vaginae. Also, it can be used for absorbing urine excreted from the urethra located between the labia like the ostium vaginae, that is, as a pad for preventing incontinence of urine.

The shape of the interlabial pad according to the embodiment may be in any shapes such as elliptic-, ovoid-, gourd-, or drop-shape as long as it is suitable to be fixed in between the labia. Also, it may have a three-dimensional structure with a projection in a convex shape or the like for tightly fixed to the labia. In order to fix it to the labia more tightly, there may be an adhesive or the like for fixing the pad to the body applied to the surface side sheet.

### [Protruded Area]

There may be a protruded area protruding towards the body side in the center of the above-described surface side sheet in the lateral direction.

In the embodiment, the finger insert direction is to be specified so that the ball of finger of the wearer is facing the surface sheet to be inserted. Thus, the concave between the labia can be precisely detected by the ball of finger (especially the fingerprint part) with a keen sense so that the protruded area with a convex shape readily provided on the surface side sheet can be appropriately fixed between the labia. Therefore, it becomes possible to increase the adhesion between the body and the interlabial pad thereby decreasing the menstrual blood leak to the outside. Furthermore, the substantial flat area other than the protruded area is fixed by being positioned to cover the pudenda thereby enabling to shield the menstrual blood flow from the lateral direction.

### [Embodiment Regarding the Size]

As shown in Fig. 3, on a garment side face 12a of a back side sheet 12, a mini sheet piece 14 covering about 2 / 3 of the above-described back side sheet 12 is attached to form a pocket 16 by bonding an outer edge 17 except for an opening for inserting a finger 19a. Specifically, the center dimension of the absorber layer 2 in the longitudinal direction is 85 mm while the center dimension of the mini sheet piece 14 in the longitudinal direction is about 55 mm. The back side sheet 12 has the area within the range of about 30 mm in the longitudinal direction, which is not covered by the mini sheet piece 14.

It is preferable that the length of the interlabial pad in the lateral direction be 10 to 60 mm and more preferable to be 30 to 50 mm. In this case, if it is longer than 60 mm, the edge of the interlabial pad and the thigh of the wearer come to be in contact thereby causing friction between them every time the wearer makes a move. Furthermore, if the friction exceeds the strength of the labia itself holding the interlabial pad, there is a risk that the interiabial pad is fallen off from between the labia. Also, if it is shorter than 10 mm, the interlabial pad cannot have the sufficient area or volume to be inserted between the labia so that the interlabial pad may easily fall off.

It is preferable that the length of the interlabial pad in the longitudinal direction be 60 to 150 mm and more preferable to be 80 to 120 mm. In this case, if it is longer than 150 mm, the contact surface between the garment side face of the interlabial pad and the underwear or the like becomes too large. Therefore, there generates the friction larger than the holding strength of the labia itself for holding the interlabial pad. As a result, the interlabial pad may fall off. Also, if it is shorter than 60 mm, the interlabial pad cannot have the sufficient area or volume to be inserted between the labia so that the interlabial pad may easily fall off.

It is preferable that the thickness of the interlabial pad according to the present invention be 0.5 to 20 mm and more preferable to be 2 to 10 mm. The interlabial pad is to be inserted between the sensitive labia so that, if the thickness is 20 mm or more, the wearer may feel the foreign feeling when wearing. On the other hand, if it is 0.5 mm or less, the capacity of the included absorber may become insufficient for absorbing menstrual blood. Thereby, there may be a case where menstrual blood is permeated through the interlabial pad.

### [Mini Sheet Piece]

The mini sheet piece 14 is attached on the above-described back side sheet 12 so as to form a pocket.

According to the embodiment, by simply bonding a mini sheet piece, which is shorter than the back side sheet in the longitudinal direction and equal in the lateral direction, with the back side sheet in the outer edge, it becomes possible to provide a gap to which a finger can be inserted with the ball of finger (especially the fingerprint part) facing the surface side sheet. Therefore, it is unnecessary to go through a specifically complicated manufacturing process in order to provide a finger insert gap so that a decrease in the productivity can be avoided.

Also, the back side sheet and the mini sheet piece are adhered in the area where the finger tip part comes to be in contact. Thus, such a case can be avoided precisely that the finger tip touches the menstrual blood at the time of wearing.

The "pocket" in this Specification means a closed member to which a whole portion of finger can be smoothly inserted. Preferably, the cross section of it is in a flat shape or something similar, however, it is not limited to these but other shapes such as semicylindrical shape may be employed.

As for the mini sheet piece, in order for the wearer to be able to easily distinguish the mini sheet piece, it can be provided to have a color, pattern, and hue different from those of the back side sheet of the interlabial pad using methods such as coloring or printing of patterns.

The above-described mini sheet piece has the length of 10 percent or more of the above-described interlabial pad in the longitudinal direction, preferably 10 to 80 percent and more preferably 30 to 60 percent.

According to the embodiment, the state at the time of inserting the finger from the opening for inserting a finger provided in the interlabial pad to the gap (finger insert gap) continued therefrom can be maintained until wearing the interlabial pad between the labia. In other words, the mini sheet piece forming the opening for inserting a finger has a specific size so that there is no chance that the finger once inserted to the finger insert gap is slipped out or the finger moves around inside the finger insert gap. Thereby, the ball of finger can be maintained facing the sheet face of the back side sheet. As a result, the state of holding the interlabial pad by the finger can be made stable and fixing in the same direction as the longitudinal direction of the pubic split can be performed more easily.

### [Bonding Position of Mini Sheet Piece]

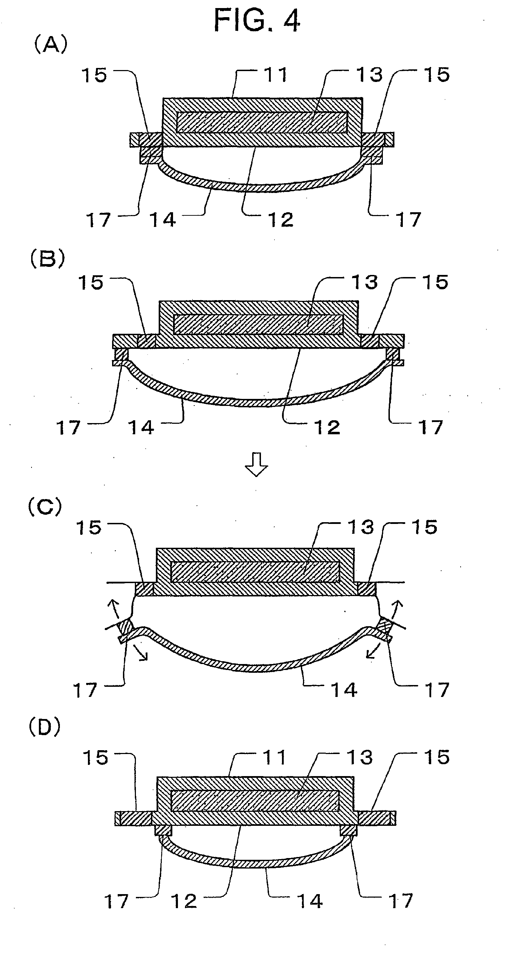

Fig. 4 is a cross section showing the cross section of the interlabial pad 1 in the lateral direction for describing the bonding state of the mini sheet piece 14. As shown in Fig. 4(A), in the case where it is fixed together by positioning the bonding area 17 in the same position as the peripheral edge 15, which is the bonding area of the surface side sheet 11 and the back side sheet 12, the outer edge 15 area becomes stiff thereby deteriorating the wear feeling. This can be avoided through fixing the mini sheet piece 14 by positioning the bonding area 17 in the area other than the peripheral edge 15.

However, as shown in Fig. 4(B), in the case where the bonding area 17 is positioned more outside than the peripheral edge 15, it is considered that friction generates due to its movement in accordance with the movements of the wearer so that there may be a case where irritation is given to the wearer as shown in Fig. 4(C).

Therefore, as shown in Fig. 4(D), it is preferable to shift the positions of the peripheral edge 15 and the bonding area 17 from each other and the bonding area 17 to be provided more inside than the peripheral edge 15.

In the preferable embodiment, the surface side sheet and the back side sheet are bonded in each of the peripheral edges. In this case, it is preferable that the absorber be not sandwiched in the peripheral edge where the sheets are bonded. To achieve this, the surface side sheet and the back side sheet are bonded so that, for example, the absorber is sealed to be in a closed state. At this time, it has to be careful so that the absorber is not sandwiched in the bonding in the peripheral edge. In this regards to this, if the absorber is sandwiched when bonding in the peripheral edge, the peripheral edge may become stiff. However, it can be avoided by providing the bonding area as described, and the preferable wear feeling is achieved. The dimension of the absorber, in order for the absorber to be not sandwiched in the above-described peripheral edge, may be the same as that of the interlabial pad or may have a smaller dimension providing a gap of 2 to 10 mm from the outer rim of the interlabial pad.

### [Finger Insert Pocket]

By inserting a finger to a pocket 16 formed by the back side sheet 12 and the mini sheet piece 14, as shown in Fig. 5, the finger can be inserted with the fingerprint surface side from the first joint up being in contact with the garment side face 12a of the back side sheet 12. Thus, as shown in Fig. 6, when leading the interlabial pad to the labia, the concave and convex of a labia 18 can be detected by having the body side face 11 a of the surface side sheet 11 being in contact with the labia so that the pad can be more precisely led inside the labia with a concave shape.

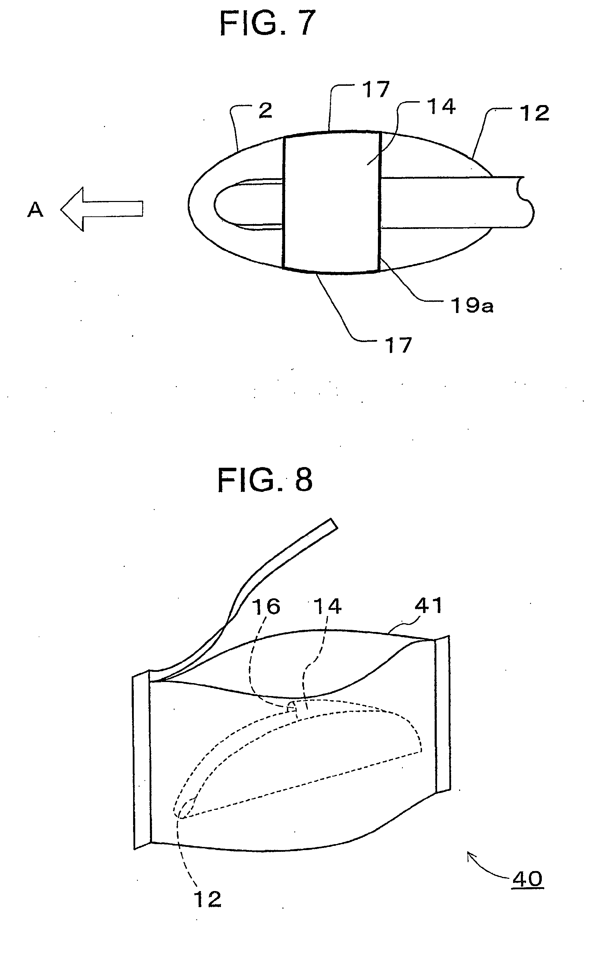

The mini sheet piece 14, as shown in Fig. 7, has the length of 10 percent or more of the absorber layer 2 in the longitudinal direction. Thereby, it can made clear that the finger insert direction is A direction. In this respect, "mini sheet piece 14 with the length of 10 percent or more" serves as the indication of the finger insert in the interlabial pad according to the present invention.

It is preferable that the above-described mini sheet piece has a stretching characteristic or elastic flexibility at least in the lateral direction of the back side sheet.

According to the embodiment, if the finger tip size of the wearer is larger than the provided opening for inserting a finger, the mini sheet piece stretches at least in the lateral direction. Therefore, regardless of the finger tip size of the wearer, the interlabial pad according to the present invention can be used effectively.

In order to provide the mini sheet piece with the stretching characteristic, a stretch spun bond nonwoven fabric can be used, whose stress is 0.1 to 0.5 N / 25 mm at the time of 5 percent stretching when being stretched at a constant testing speed of 100 mm / min with the grip interval of 100 mm.

On the other hand, in order to provide the mini sheet piece with the elastic flexibility, a fabric sheet or a film sheet in which a thermoplastic elastomer resin is used may be used. Also, an elastic flexible material such as the thermoplastic elastomer resin or natural rubber may be used alone or may be used by mixing it with a non-elastic flexible material.

The whole girth of the inside of the above-described opening for inserting a finger may be 30 to 100 mm, and more preferably to be 40 to 80 mm.

In the case where the whole girth of the inside of the above-described opening for inserting a finger is shorter than 30 mm, the opening for inserting a finger itself becomes small thereby causing a trouble when putting a finger in and out. On the other hand, if it is longer than 100 mm, the interlabial pad cannot be fixed to the finger. Therefore, it becomes difficult for the ball of finger to be surely in contact with the sheet surface thereby causing a trouble when wearing.

### [Individual wrapping container]

In the present invention, an individual wrapping container for covering the whole portion of the interlabial pad is used. The individual wrapping container has a sealing property in which microbes or contamination hardly enter. In the individual wrapping container, the interlabial pad is positioned in such manner that the interlabial pad is easily worn upon opening the opening of the individual wrapping container. In other words, it is preferable to have a structure in which the interlabial pad can be easily taken out from the individual wrapping container without a contact with the surface side sheet of the interlabial pad by using a form (for example, mini sheet piece) in which the user can easily achieve wearing.

Specifically, in the case where the interlabial pad has a finger-cot shape to which a finger can be inserted, a finger can be inserted and fixed with the inside of the finger tip facing the back side sheet of the interlabial pad upon opening the individual wrapping container. The interlabial pad can be taken out from the individual wrapping container while being fixed to the finger tip. In short, the opening of the individual wrapping container stands in the same direction as the finger tip insert direction of the interlabial pad in which the number of live microorganism is suppressed and is wrapped in the individual wrapping body.

When using a gas disinfection method as a disinfection method, at least a part of bonding area of the individual wrapping container is adhered in dots or the like thereby providing a breathing characteristic. Thereby, the interlabial pad inside the individual wrapping container can be disinfected.

Next, the individual wrapping body wrapping the interlabial pad to which the mini sheet piece for finger insert is attached will be described by referring to Fig. 8 to Fig. 16.

In an individual wrapping body 40 for wrapping the interlabial pad according to the embodiment, the interlabial pad is folded in two and the mini sheet piece 14 is positioned to be near an opening 41. The pocket 16 for finger insert is also positioned near the opening 41 and, in addition, the interlabial pad 1 is wrapped facing in a different direction of the individual wrapping body 40 so that the pocket 16 naturally opens. Therefore, upon opening, an opening for inserting a finger 19a is opened. Thus, a wearer can insert the finger immediately with the ball of finger being in contact with the back side sheet 12.

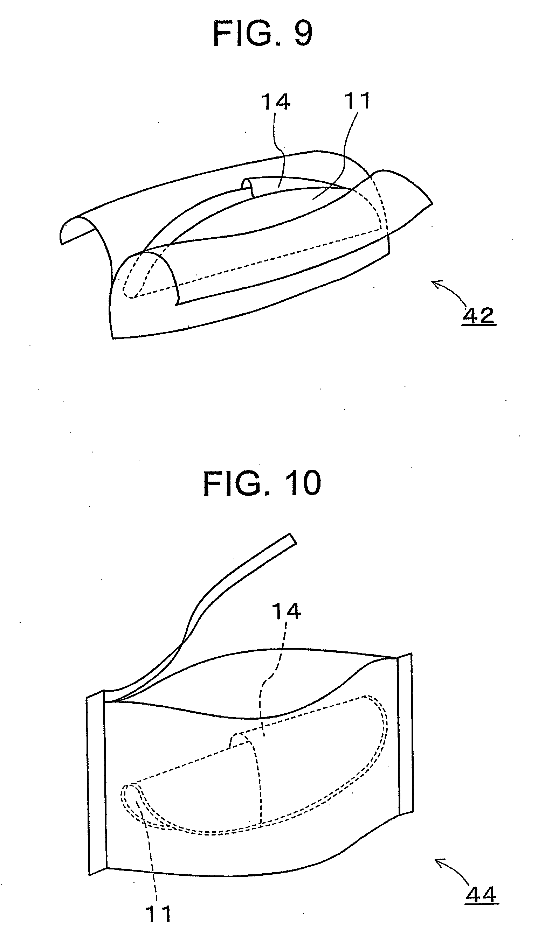

The individual wrapping body, for example, may be an individual wrapping body 42 in a form of a set of folded doors as shown in Fig. 9 or an individual wrapping container 44, as shown in Fig. 10, which is opened by stripping off the top part as long as the finger of the wearer can be inserted to the pocket provided in the inter labia pad 1 immediately after opening. As shown in Fig. 9, the pad may be folded in two along the longitudinal direction with the surface side sheet 11 being the outer side or, as shown in Fig. 10, or may be folded in two along the longitudinal direction with the surface side sheet 11 being the inner side.

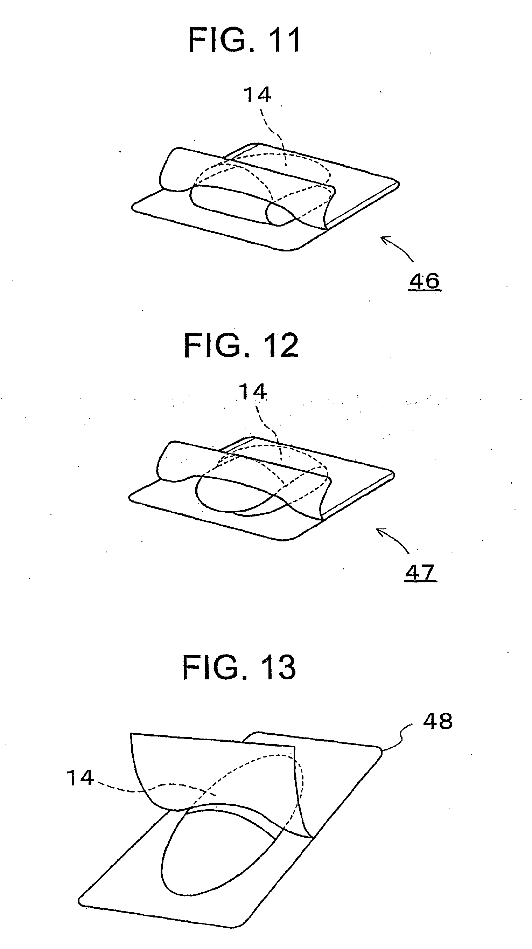

It may be an individual wrapping body 46 or 47 as shown in Fig. 11 and Fig. 12, which is opened by stripping off the top part. In Fig.11 and Fig. 12, the interlabial pad is folded in two in the lateral direction. Fig. 11 shows the case where a shallow pocket is provided and Fig. 12 shows the case where a deep pocket is provided. Also, when it is folded in the longitudinal direction, an opening may be provided in the individual wrapping body and the interlabial pad may be taken out. Furthermore, as shown in Fig. 13, it may not be folded at all. The shape of he interlabial pad wrapped inside when being wrapped is not specifically limited as long as it can be wrapped in the state where a finger can be inserted upon opening.

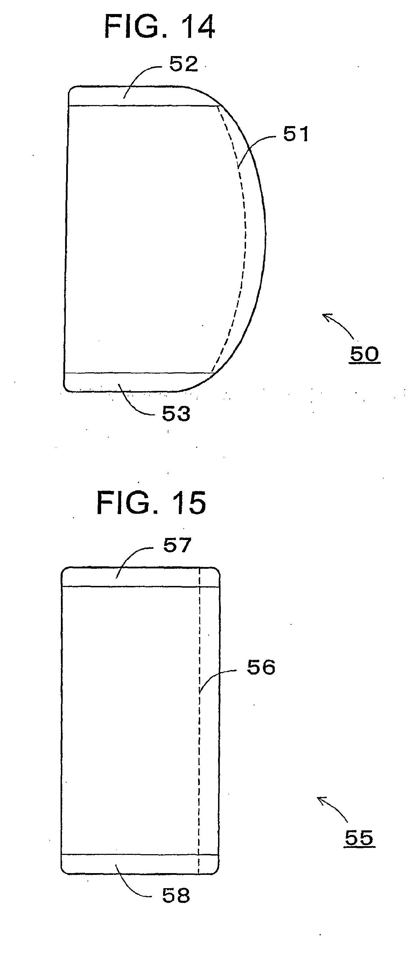

Also, the interlabial pad, as shown in Fig. 14, may be folded in two along the longitudinal direction to be enclosed and the four sides may be sealed. Or it may be left open along the stitches 51 in the arc. Peripheral edges 52 and 53 are bonding areas by embossing, hot melt adhesive or the like. On one side of the individual wrapping container, where there is a back side sheet (finger tip insertion opening) of the interlabial pad, a breaking surface (stitched) which is selectively broken by outer pressure is applied. Thereby, cut can be easily opened.

In Fig. 15, an individual wrapping container 55 has a substantial quadrilateral shape and the interlabial pad is folded in two in the longitudinal direction to be enclosed. There are stitches on the peripheral edge 56 in the longitudinal direction. The peripheral edges 57 and 58 in the lateral direction are bonded by embossing, hot melt adhesive or the like.

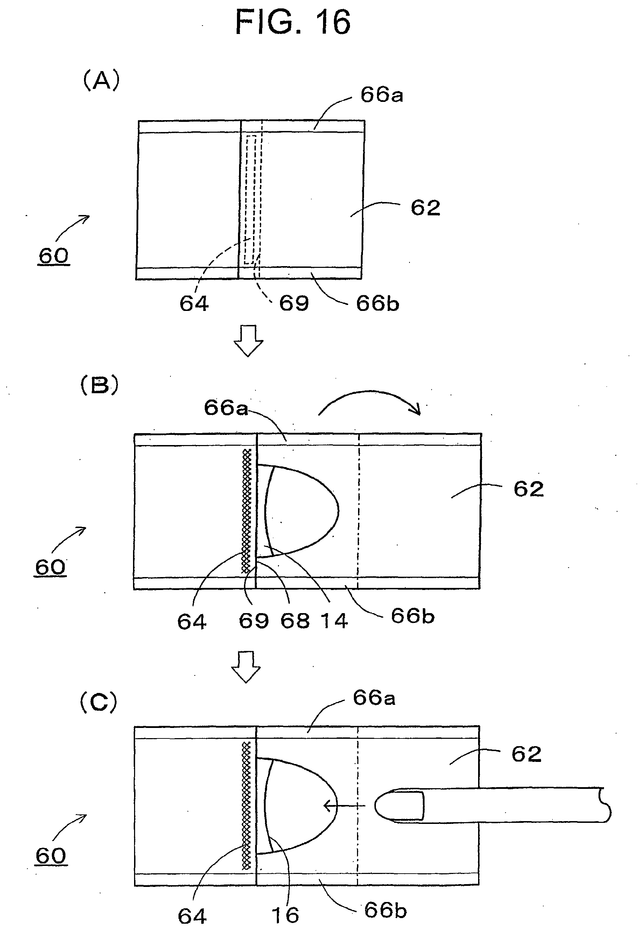

Fig. 16 shows an individual wrapping container 60 made of a polyethylene film. As an opening other than providing stitches, for example, as shown in Fig. 16, an adhesive capable of re-sealing can be used as well. Fig. 16 (A) shows the state where a lid 62 of the individual wrapping container is folded. The lid 62 covers over an edge 69 of a pocket 68 of the individual wrapping container. The lid 62 is fixed by a hot melt 64 capable of re-sealing to be capable of putting on and off. Edges 66a and 66b in the longitudinal direction are bonded by dotted pattern embossing. The interlabial pad is in the pocket 68 of the individual wrapping container 60 and the top part of the mini sheet piece 14 sticks out from the edge 69 of the pocket of the individual wrapping container 60. By inserting a finger in the pocket 16 of the mini sheet piece 14, the interlabial pad can be taken out. The pocket of the individual wrapping container 60 is formed by folding a polyethylene film and boding the edges 66a and 66b in the longitudinal direction.

For the individual wrapping container, not only the polyethylene sheet but also the thermoplastic sheet such as olefin type resin or polyester type resin can be used. Also, a sheet to which a nonwoven fabric is laminated may be used.

For the inner surface side of the individual wrapping container, with the consideration of the smooth texture, it is preferable to be formed by using nonwoven fabrics. Examples of such nonwoven fabrics are crape tissue with a specific weight per unit area of 15 to 50 g / m², a wet spun lace nonwoven fabric with a specific weight per unit area of 15 to 70 g / m², which is a mixture of cotton and pulp in which at least 10 percent by mass or more of cotton is contained, a spun lace nonwoven fabric containing at least 30 percent by mass or more of rayon with a specific weight per unit area of 20 to 70 g / m², and a melt blown nonwoven fabric formed of PP with a specific weight per unit area of 20 to 50 g / m². Also, it may be formed using a composite nonwoven fabric provided by sandwiching a melt blown nonwoven fabric with a specific weight per unit area of 5 to 20 g / m² by a spun bond nonwoven fabric with a specific weight per unit area of 6 to 10 g / m². On the other hand, for the outer surface side of the wrapping container, with the consideration of water-pressure resistance, it is preferable to be formed of a film made of PE with a specific weight per unit area of 10 to 30 g / m², a perforated plastic film with the perforation rate of 10 to 30 percent and a specific weight per unit area of 15 to 30 g / m².

The inner surface side material and the outer surface side material of the above-described individual wrapping container are formed to be one body by a laminate processing using a method well known to those skilled in the art such as a hot melt adhesive, heat embossing and ultrasonic sealing. At this time, in the case of using a hot melt adhesive, it is preferable to apply the adhesive in spiral or lines with the application amount of 3 to 10 g / m² and the application face ratio of 5 to 40 percent. In the case of using heat embossing or ultrasonic sealing, it is applied in the arrangements of lines, dots, or cross-lines, with the sealing surface ratio of 5 to 20 percent. It is to consider the texture of the laminated material.

Also, with the consideration of the convenience for the users, it is preferable to be able to flush down the individual wrapping container after taking out the interlabial pad. The preferable materials to be used are a film sheet of polyvinyl acetate (PVAC), polyvinyl alcohol, methylhydroxypropylcellulose (MHPC) or the like, a water dispersible nonwoven fabric obtained by laminating these materials, a tissue, and a water soluble paper.

The individual wrapping body may be formed of a biodegradable material and *l* or a water soluble material and / or a water dispersible material.

If the individual wrapping container is formed of a water soluble material or a water dispersible material, the individual wrapping container can be flushed down to the toilet. Therefore, wearers can be freed from the complication of discarding the individual wrapping container and a decrease in the trashes in the toilet can be achieved at the same time.

Specific examples of such individual wrapping container are a composite material obtained by laminating tissue with a specific weight per unit area of 15 to 40 g / m² and polyvinyl alcohol with a specific weight per unit area of 20 to 50 g / m², and then applying 0.5 to 1 µm of silicone on the polyvinyl alcohol side, and a spun bond nonwoven fabric with a specific weight per unit area of 15 to 40 g / m² using poly lactic fiber as the main component.

### [Package]

Provided is a package capable of enclosing and disinfecting the interlabial pad sealed inside the individual wrapping container. The structure of the package is not specifically limited as long as it comprises a main body of the container and a lid with a structure to which contaminations cannot enter from outside into the inside of the container before being opened and the lid is capable of covering the opening of the container even being repeatedly opened and closed after opening the package so that entering of contaminations can be decreased as much as possible. It is not necessary that the main body of the container and the lid are formed separately but they may be formed continuously.

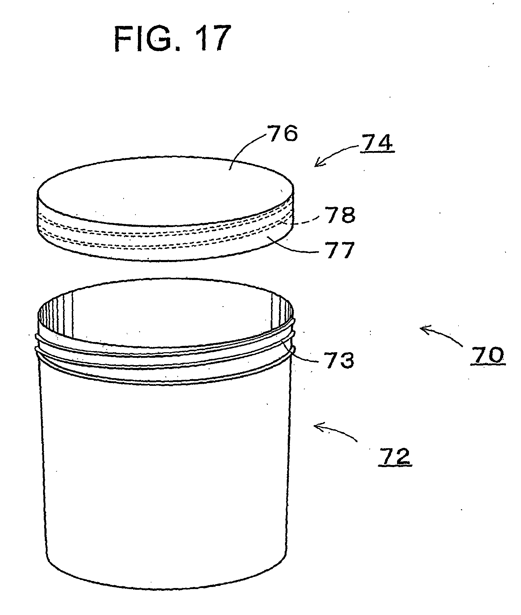

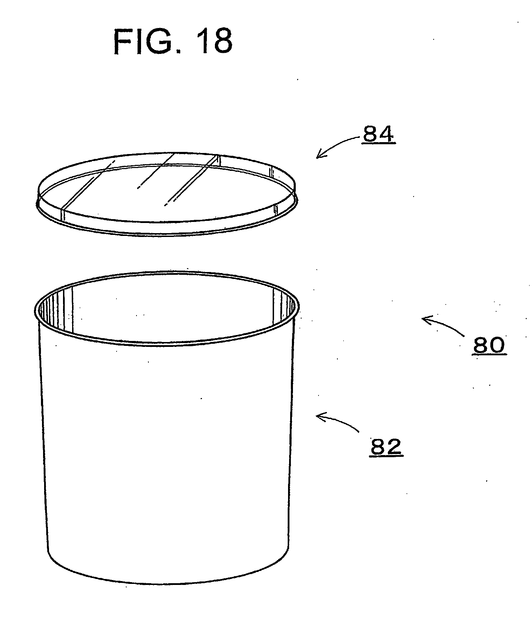

To specifically describe the container which can be used by being repeatedly opened and closed, it is preferable to comprise a main body 72 of the container and a lid 74. The lid 74 comprises a top face 76 and a side face 77 which stands from the peripheral edge of the top face. It may have a screw cap structure (Fig. 17) in which screw grooves 73 and 78 are provided in the main body 72 of the container and the lid 74. It may also be a poly cap structure (Fig. 18) in which a soft lid 84 is repeatedly used for a hard main body 82 of the container.

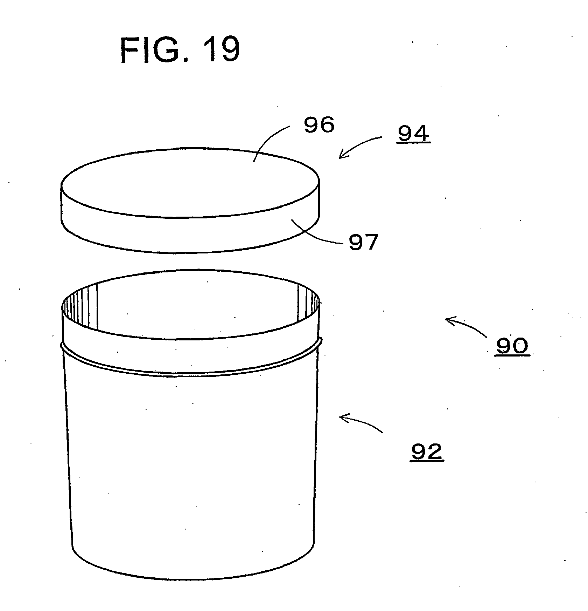

It may also be a polypropylene mold cap method (Fig. 19) or the like which comprises a main body 92 of the container with an opening and a lid 94 comprising a top face 96 and a side face 97 which stands from the peripheral edge of the top face.

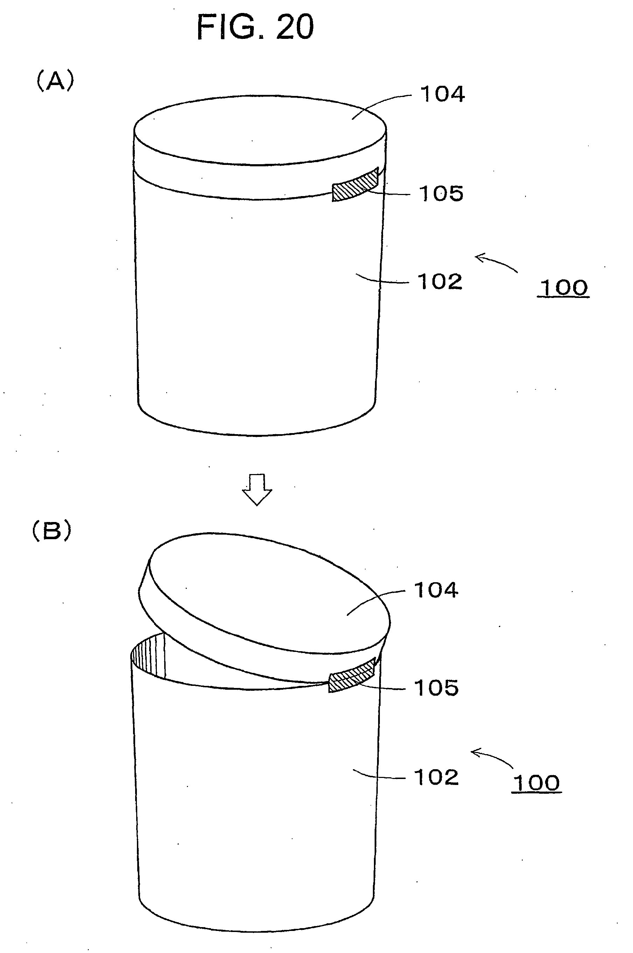

The preferable embodiment is as shown in Fig. 19. A container 102 and a part of a lid 104 are bonded by a bonding member 105 within the range capable of opening and closing. Therefore, it is possible to surely cover the opening of the container (Fig. 20) using the lid in regards to simple operation for the user and a repeated operation of opening and closing.

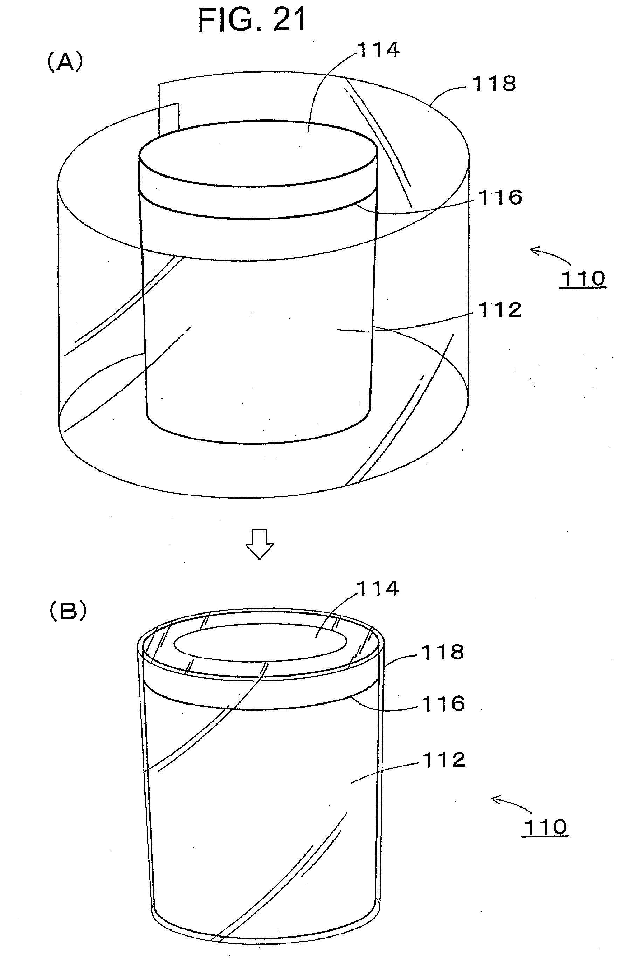

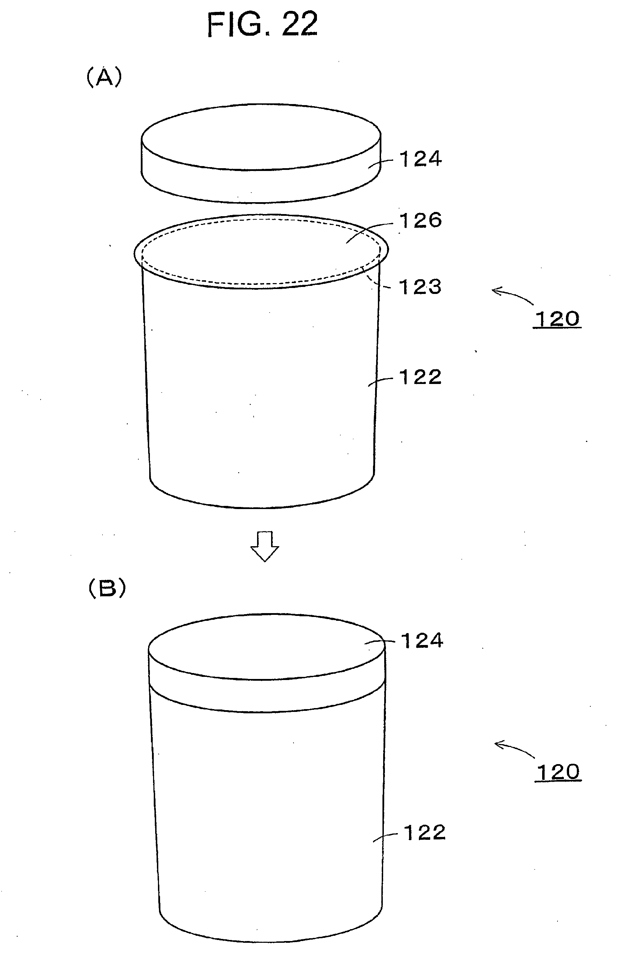

The structure to which contaminations can boundlessly hardly enter from outside into the inside the container before being opened will be described. Examples of such structure are a structure (Fig. 21) in which a gap 116 generated between a main body 12 of the above-described container and a lid 114 is over-wrapped by a shrink film or the like, or a structure (Fig. 22) in which a seal member 126 capable of covering the whole portion of the opening is adhered to a main body 122 of the container in the peripheral edge 123 and the main body 122 of the container is covered by the lid 124.

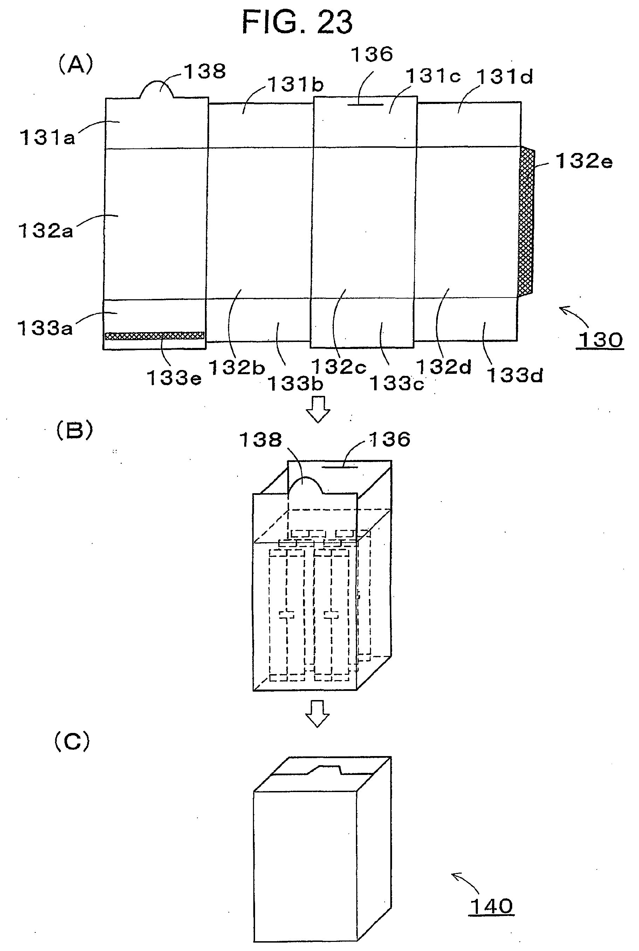

Also, the package, as shown in Fig. 23, may be formed by composing a cardboard or the like. Fig. 23 (A) is an unfolded view of a rectangular container and the main body of the container comprises side faces 132a, 132b, 132c, 132d and bottom faces 133a, 133b, 133c, and 133d. The lid comprises top faces 131a, 131b, 131c, and 131d. An application face 132e to which an adhesive is applied is formed continuously from the side face 132d and an application area 133e to which an adhesive is applied is formed on the bottom face 133a. A cut 136 is provided on the top face 131c, and a projection 138 corresponding to the cut is formed on the top face 131a.

A composed body shown in Fig. 23(B) can be achieved by folding the unfolded figure and then adhering the application face 132e to the inner side of the side face 132a and the application area of the bottom face 131 a to the bottom face 131c. A plurality of individual wrapping body can be enclosed in the composed body.

In Fig. 23(B), by folding the top faces 131a, 131b, 131c, and 131d and inserting the projection 138 of the top face 131 a to the cut 136 of the top face 131c, as shown in Fig. 23(C), the package is completely composed.

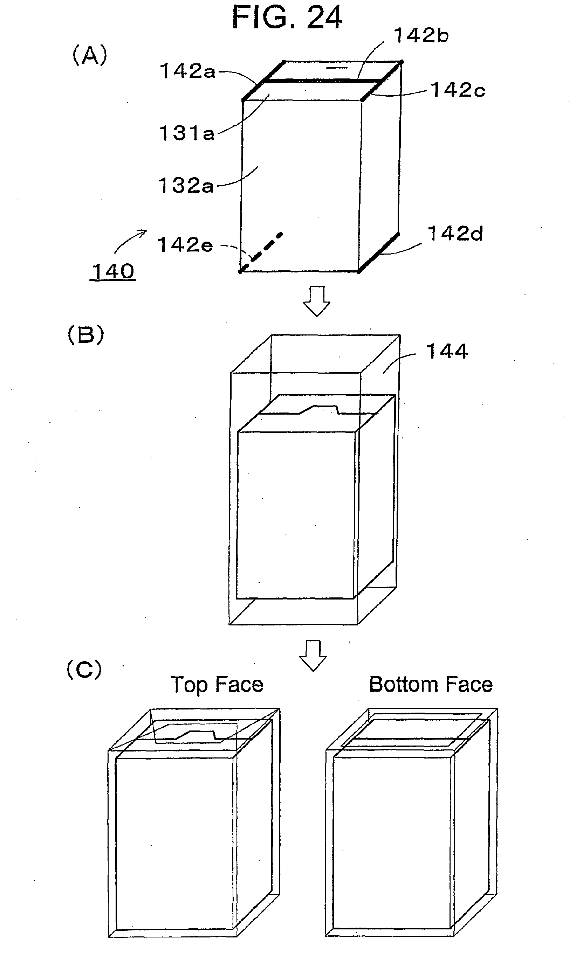

In the package, as shown in Fig. 24(A), there are gaps 142a, 142b, 142c, 142d, and 142e. In order to fill the gaps, a shrink film may be applied for over-wrapping. As shown in Fig. 24(B), by covering the package with a shrink film 144, over-wrapping can be achieved as shown in Fig. 24(C).

### [Disinfetion Processing, Especially Sterilization]

In the final state where the interlabial pad is enclosed in the individual wrapping container and packed in the package, the interlabial pad may be sterilized. Alternatively, the interlabial pad may be sterilized before being enclosed in the individual wrapping container and packed in the package.

Specifically, it can be sterilized by bringing it to a contact with ethylene oxide gas which is broadly used for sterilizing tampons which are medical goods. After sterilizing the interlabial pad by bringing it to a contact with ethylene oxide gas at the temperatures within the range of 30 °C to 70 °C, ethylene oxide gas is removed from the interlabial pad. As for the removal, after decreasing the pressure until the inner pressure becomes -91.2 kPa or less, air is inserted for recovering the pressure to the atmospheric pressure. This operation is performed once or more. Or after decreasing the pressure to the range within - 40 kPa to -91.2 kPa, it is allowed to remain at the temperatures within the range of 50 °C to 60 °C for 5 to 20 hours, and then it is recovered to the atmospheric pressure.

There are methods described below as the method of sterilization, that is, for exterminating or removing all of the microorganism. However, it is not specifically limited to these as long as it is possible to perform sterization without influencing the interlabial pad.

Considering the toxic characteristic of the ethylene oxide gas used in a gas sterilization processing, a sterilization using hydrogen peroxide or ozone may also be used. It is more preferable to employ an electron ray irradiation sterilization method capable of performing sterilization in which sterilization is performed in a short period of time even if it is a sealed wrapping.

For example, irradiation method or radiation method may be used. The radiation method is a method in which microbes are perished by irradiating radiation such as gamma radiation from the source including radioactive isotope element. Examples of irradiation method also include an ultraviolet method, a high frequency method and an electron ray method. The ultraviolet method is a method for perishing the microbes by irradiating ultraviolet rays. The high frequency method is a method for perishing the microbes through the heat generated by directly irradiating the high frequency. The electron ray method is a method for perishing the microbes by irradiating the electron rays.

Gassing is a method for perishing the microbes using a gas such as ethylene oxide, formaldehyde or hydrogen peroxide.

### [Material for Surface Side Sheet]

For the surface side sheet, it is preferable to select the material, which is permeable and gives no stimulation to skins. For example, the materials can be used are a nonwoven fabric obtained by the manufacturing methods such as melt blown, spun bonding, through-air, point bonding, needle punching, spun lacing and the like. However, considering the contact ratio between the inner wall of the labia, it is preferable to use a single nonwoven fabric obtained by the manufacturing method such as spun lacing, melt blown, and needle punching, or to use some of them being combined together.

It is also preferable to use a sheet obtained by mixing, or a filament selected from the group comprising rayon, acetate, natural fibers such as cotton and pulp, a filament made of a synthetic resin or a composite fiber with a sheath-core structure, a synthetic fiber to which a hydrophilic processing is performed. Specifically, an example can be prepared in the following manner. The fiber in which the proportion of 10 to 1 percent by mass of synthetic fiber, 4 to 30 percent by mass of natural cotton, 60 to 95 percent by mass of rayon or acetate are blended and is prepared to have a specific weight per unit area of 20 to 50 g / m². Subsequently, the fibers are entangled to each other by water-flow interlacing treatment and then dried to prepare spun lace nonwoven fabric with the thickness of 0.1 to 1.0 mm.

Synthetic fiber is blended with the above-described spun lace nonwoven fabric so that the bulkiness and the distance between the fibers can be easily maintained even if the surface side sheet comes to be in contact with menstrual blood and becomes wet. The blending proportion of the synthetic resin is kept relatively small so that the fiber rigidity is maintained even if it becomes wet by menstrual blood and, if it is unnecessarily large, the inner wall of the labia may be harmed by the high fiber rigidity. It is preferable that the synthetic fiber is selected from the group comprising filament such as polypropylene (PP), polyethylene (PE), polyethylene terephthalate (PET), fiber formed of a graft polymer of PE and PP, a compound synthetic fiber with a sheath-core structure in which the core is PP or PET and the sheath is PE, an eccentric-type sheath-core structure and side-by-side structure. Also, it is preferable to use it by making it milky by mixing a filler made of titanium oxide, calcium carbonate and the like within the range of 0.5 to 10 percent by mass if necessary.

The fiber used for the above-described spun lace nonwoven fabric is selected from natural cotton with the fiber length of 15 to 60 mm, rayon or acetate with the fiber length of 25 to 51 mm, and the fineness of 1.1 to 6.6 dtex. In this case, if fiber with a high specific surface area is used, the contact area between the inner wall of the labia can be increased. Thus, it is preferable that falling off of the pad from the labia can be decreased. For example, it is preferable to use rayon or acetate whose cross sectional shape of the fiber is a variant cross sectional shape such as Y-shape or C-shape. By providing the cross sectional shape to be a variant cross sectional shape, not only the specific surface area is increased compared to that of a complete circle but also the gap between the fibers are increased. Therefore, the rigidity of the surface side sheet is decreased so that the adhesion between the inner wall of the labia is improved. Accordingly, it is preferable that the risk of falling-off the pad from the labia and menstrual blood leak can be decreased.

Another preferable example of the surface side sheet is a spun lace nonwoven fabric obtained by stretching a spun lace nonwoven fabric with a specific weight per unit area of 20 to 50 g / m² in which 15 to 5 percent by mass of synthetic fiber, 50 to 10 percent by mass of natural cotton, and 35 to 85 percent by mass of rayon or acetate are blended and then is stretched about 10 to 80 percent in the width direction and also stretched about 10 to 80 percent in the longitudinal direction. The spun lace nonwoven fabric has a high compressional gradient and the entanglement between the fibers is once loosened in plat state. Therefore, each of the fibers, specifically, the synthetic fiber with high fiber rigidity performs spring back. The spun lace nonwoven fabric has fibers with a substantial loop shape projected towards inner wall of the labia.

The projected substantial loop shape fibers can buffer the friction drag between the inner wall of the labia and the pad surface in the shearing direction. Therefore, not only decreasing the risk of damaging the inner wall of the labia but also the speed of menstrual blood running from the substantial flat shape inner wall of the labia to the underwear direction can be decreased. Thus, menstrual blood can be easily transmitted to the absorber inside the pad.

The height and pitches of the projected substantial loop shape can be controlled by changing the expanding ratio or the stretching ratio of the nonwoven fabric, or changing the entangling strength between the fibers by the fabricating method of the nonwoven fabric. It may also be controlled by using a compound synthetic fiber with eccentric-type sheath-core structure and side-by-side structure as the synthetic fiber and adjusting the winding reduction rate of each fabric filament using the difference in heat shrinkable rate of the resin.

Still another preferable example of the surface side sheet is a perforated film obtained by performing perforation, thermal embossing or the like on a thermoplastic film, and a compound sheet of the perforated film and a nonwoven fabric. Especially, a sheet is preferable in which number of micro projections with standing fibers are provided by applying water jet processing to the nonwoven fabric part of the above-described compound sheet. The surface drag generated by the above-described projections decreases the flowing speed of menstrual blood on the surface of the surface side sheet. Thereby, it becomes possible to surely absorb the menstrual blood without flowing it down. The height of the projection and the distance between the projections are preferable to be 0.1 to 4 mm. If it is below the range, the gap by which menstrual blood enters inside the pad becomes small so that it is difficult to surely absorb the menstrual blood. If it exceeds the range, the projection may be collapsed due to the body pressure and the like at the time of wearing. Therefore, both cases are not preferable.

A part of, or the whole portion of the surface side sheet may be perforated and the perforation rate is preferable to be 3 to 30 percent. If it is less than the range, the effect of transmitting the menstrual blood to the absorber side is small. If it exceeds the range, the contact ratio between the inner wall of the labia is decreased so that the pad may fall off from between the labia. Therefore, both cases are not preferable.

Among the materials, considering the liquid mobility from the inner face of the labia, chemical stimulation by an activator, and adhesion with the inner wall of the labia, it is preferable to laminate rayon with 1.1 to 4.4 dtex fineness and 7 to 51 mm fiber length by 40 to 80 percent of a total specific weight per unit area on the body surface side, and to laminate a mixture of rayon with 1.1 to 4.4 dtex fineness and 7 to 51 mm fiber length by 14 to 42 percent of a total specific weight per unit area and PET with 1.1 to 4.4 dtex fineness and 7 to 51 mm fiber length by 6 to 18 percent of a total specific weight per unit area on the garment face side. After laminating them so that the total specific weight per unit area of the two layers becomes 20 to 60 g / m², the fibers are entangled by water-flow interlacing treatment and then dried to prepare spun lace nonwoven fabric with the thickness of 13 to 0.50 mm. The spun lace nonwoven prepared as described is preferable. At this time, by mixing PET on the garment side, bulkiness can be easily maintained even if the permeable sheet becomes wet. Therefore, adhesion between the inner wall of the labia can be maintained.

### [Material for Back Side Sheet]

As for the material used for the back side sheet, in the case of using a permeable material, the same material as that of the surface side sheet can be used. In this case, it is preferable to use the pad together with a sanitary napkin (pad for use with a sanitary napkin).

In the case where an impermeable material is used for the above-described back side sheet, leaking out of the menstrual blood absorbed in the absorber to the outside of the interlabial pad can be prevented. Furthermore, by providing it with a material for moisture tranceparency, stuffiness can be decreased when wearing thereby enabling to decrease the discomfort when wearing.

When using an impermeable material, preferably used are an impermeable film in which synthetic resin such as PE or PP is provided to be thin, a breathing film obtained by filling an inorganic filler to a synthetic resin and then applying a stretching processing, a laminate film obtained by combining a paper / a nonwoven fabric and an impermeable film, a material obtained by bonding a breathing resin film with the back side of a nonwoven fabric such as spun bond or spun lace to which a repellent processing is applied. An example of the method for providing breathing property to the impermeable sheet is a method in which capillaries with 0.1 to 0.6 mm diameter are formed for the absorber by perforation rate of 10 to 30 percent.

More specific structural example when using the impermeable material is a film mainly made of low density polyethylene (LDPE) with the density of 0.900 to 0.925 g / cm³ and a specific weight per unit area of 15 to 30 g / m². It is to consider the softness so that the wear feeling cannot be deteriorated.

It is more preferable to reduce the contact ratio to decrease the friction drag value by embossing the above-described film to provide convex-shaped projections in order to, when the pad is fixed between the labia, decrease the risk of the interlabial pad from being fallen off from the labia due to the high friction caused by the contact between the impermeable sheets, or between a pad used together, an underwear or the like.

### [Material for Absorber]

It is preferable for the absorber to be bulky, hard-to-be deformed, and less chemically stimulant, although any material can be used as long as it is capable of absorbing and holding liquid (menstrual blood). For example, the material may be prepared by properly blending ground pulp, rayon, acetate, natural cotton, air-laid pulp to which chemical bonding is performed, highly absorptive polymer, highly absorptive polymeric fiber, and a synthetic fiber. Also, the same sheet as that of the above-described surface side sheet may be used.

An example of the preferable absorber is a nonwoven fabric sheet obtained by mixing and laminating 60 to 90 parts by mass of rayon or acetate with fineness of 1.1 to 6.6 dtex and 10 to 40 parts by mass of highly absorptive polymeric fiber, and then entangled and formed to be a sheet by needling, with a specific weight per unit area of 50 to 250 g / m² and the bulkiness of 2 to 5 mm. When this nonwoven sheet is enclosed inside the pad, it is possible to adjust the bulkiness by stacking or folding when necessary.

It is preferable for the absorber, although any material can be used as long as it is capable of absorbing and holding liquid (body fluid), to be bulky, hard - to - be deformed, less chemically stimulant, and highly flexible to fit into the labia. Specifically, a nonwoven sheet in which, 50 to 150 g / m² of pulp selected from the range of the fiber length of 1 to 10 mm is laminated on the garment face side and, on the body surface side, 150 to 250 g / m² of a mixture obtained by mixing 60 to 90 percent of rayon with 1.1 to 4.4 dtex fineness and 20 to 51 mm fiber length with 40 to 10 percent of natural cotton by this mixing ratio is laminated, which then to be formed into a sheet by dotted embossing to have 2 to 10 mm bulkiness, and more preferable to have 3 to 5 mm bulkiness. Thereby, liquid can be easily transmitted from the body surface side to the garment surface side resulting in the improvement of the absorbing and holding capacity. Furthermore, by providing a mesh spun lace nonwoven fabric of rayon with 1.1 to 4.4 dtex fineness and 25 to 51 mm fiber length by a specific weight per unit area of 15 to 40 g / m², the liquid transmitted from the body surface side can be dispersed by the mesh spun lace to be induced to almost all over the region of the pulp layer. Therefore, more liquid can be effectively absorbed.

### [Material for Mini Sheet Piece]

The material used for the mini sheet piece is not specifically limited as long as it has a sheet structure such as a fabric, a nonwoven fabric, or a plastic sheet. However, it is preferable to be a sheet structure in which surface contact area is decreased by surface concave-convex processing such as embossing, application of pleats or craters. Also, it is preferable that the sheet structure has a stretching characteristic or flexibility in the lateral direction.

Examples of the material forming the mini sheet piece are natural fibers such as cotton, silk, and hemp, examples of the regenerated fibers are regenerated cellulose fiber, rayon, cuprammonium rayon, and examples of synthetic fibers are polyolefin containing fiber, polyester system fiber, polyamide containing fiber (nylon or the like), polyvinyl alcohol containing fiber, polyacrylonitrile containing fiber, and polyurethane.

Especially, when the mini sheet piece is formed of the nonwoven fabrics, web forming can be performed either by dry method (carding, spun bonding, melt-blown, air-laid and the like) or wet method, or a plurality of the methods may be combined to be used. Examples of bonding methods are thermal bonding and needle punching. However, it is not specifically limited and spun lace formed by water-flow interlacing treatment can be also used preferably.

When forming the mini sheet piece using the plastic sheet, a sheet of thermoplastic resin (PE, PP, PET, polylactate, polybuthylene succinate and the like), and a perforated foamed material and the like can be used. As the method for providing stretching characteristic or flexibility to the mini sheet piece in the lateral direction, the mini sheet piece may be formed of a fiber sheet or film sheet using thermoplastic elastomer resin. Also, it may be formed using a material obtained by combining the non-elastic flexible material and the thermoplastic elastomer resin or the elastic flexible material such as natural rubber.

### [Adhesion Area]

In order to further decrease the risk of the pad from falling off from between the labia, it is preferable to form an adhesion area by applying adhesive on the surface covering the surface side sheet. By attaching the adhesion area near the labia of the wearer, the risk of falling off is decreased.

Examples of the manner in which the adhesive is applied are in the form of whole surface application or in dots, mesh, or lines. The application position of the adhesive agent is not specifically limited as long as it enables fixing of the pad to the body. However, specifically considering the existence of the pubic hair-grown part in the area in front of the labia, it is preferable to apply the adhesive near both end sides of the inter labia pad in lines in about 1 to 5 mm width.

"Adhesion area" can be formed by applying adhesive on the surface side sheet. As the adhesive agent which can be used in the present invention, a gel adhesive made of water soluble polymer, a crosslinking agent, a plasiticizer and moisture can be used. More specifically, examples of the water soluble polymer used herein is gelatin, polyacrylic acid sodium, polyvinyl alcohol, and carboxymethyl cellulose. Examples of the crosslinking agent are water soluble metallic salt such as calcium chloride and magnesium salfate and examples of the plasticizer are glycelol, wax, and paraffin.

As other adhesive agent, so-called a pressure sensitive hot melt can be also used as the adhesive agent for forming the adhesion area. Incidentally, the pressure sensitive hot melt is mainly formed of synthetic rubber resin such as styrene-isoprene-styrene block copolymer (SIS), styrene-butadiene-styrene block copolymer (SBS), styrene-ethylene-butadiene-styrene block copolymer (SEBS), and styrene-ethylene-propylene-styrene block copolymer (SEPS). The pressure sensitive hot melt adhesive can be obtained by fusing and mixing the tackifier such as terpene resin or rosin resin and a plasticizer such as wax to the pressure sensitive hot melt.

Also, silicone adhesive agent can be used as other adhesive. An example of the silicone adhesive is a mixture obtained by mixing silicone resin or fluorocarbon resin with a crosslinking agent such as metallic salt of platinum, molybdenum, or antimony and a plasticizer such as ester wax, glycerin, or machine oil.

As described, there are many kinds of adhesives for forming the adhesion area. However, if the application stability is taken into consideration, the pressure sensitive hot melt is preferable. An example of the pressure hot melt adhesive with a high application stability is the one which can be prepared by fusing and mixing 15 to 25 percent by mass of SEBS, 15 to 35 percent by mass of plasticizer, and 40 to 70 percent by mass of tackifier. An antioxidant, antifluorescent or the like may be added to the pressure sensitive hot melt within the range of 0.1 to 1.0 percent by mass.

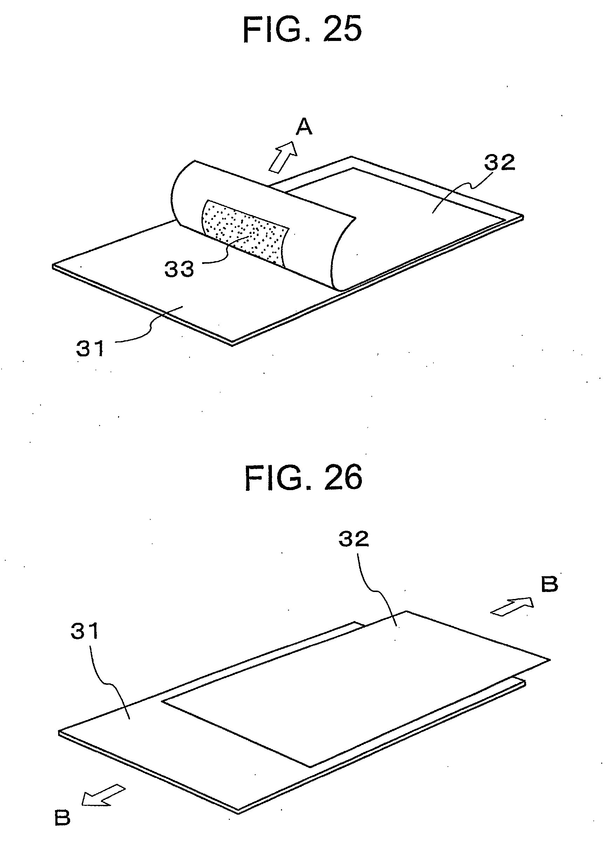

An example of valuation method of the adhesive strength will be described in detail. The valuation method is to measure the separation force (Fig. 25) and the shearing force of the adhesive (Fig. 26). It is carried out using a constant speed expansion tensile tester and a stainless plate 31 of 80 mm in length × 50 mm in width. As a preparation for the evaluation test, a test piece of a polyethylene film 32 having substantially same size as the stainless plate 31, in which an adhesive 33 is applied within the range of 25 mm in width and 50 mm in length, is left for 30 minutes at a room temperature (20 °C) beforehand. Subsequently, the polyethylene film 32 is put lightly over a stainless plate 31 so that the adhesive 33 comes to be in contact with the stainless plate 31, and a roller is applied once (one way only) at 30g / cm² pressure. Then, it is left for 30 minutes at a room temperature (20 °C) to fabricate a test piece.

The polyethylene film 32 part of the test piece obtained as described is used. In the separation force test of the adhesive, it is separated in the pulling direction of arrow A in Fig. 25 (180 ° separation) and, in the shearing force test of the adhesive, it is pulled in the pulling direction of arrow B in Fig. 26. The test condition is provided to be 100 mm / min testing speed.

In the case where the forces are measured by the measurement method described above, it is preferable that the measurement value of the separation force be 100 to 2000 mN / 25 mm and that of the shearing force be 3000 to 15000 mN / 25 mm, in consideration of the burden imposed on the skin of the wearer.

It is preferable to cover the part where the adhesive is applied with a sheet which is obtained by coating silicone resin on a tissue paper, which is a generally obtainable separate paper, or a sheet obtained by coating silicone resin on a film. Thereby, damages or separation of the adhesive part can be prevented while being preserved.

### [Structure of Interlabial Pad to Which Biodegradability, Water Dispersibility, Water Solubility are Provided]

It is preferable that the interlabial pad according to the present invention be formed of a biodegradable material and / or a water soluble material and / or a water dispersible material. Such pad can be dropped and flushed down to the toilet. Therefore, the pad can be easily and sanitarily discarded and trashes in the toilet can be further decreased at the same time.

In this Specification, "biodegradability" means that a substance is decomposed into gas such as carbon dioxide or methane, water, and biomass under anaerobic or aerobic condition according to the natural process under the existence of bacteria represented by actinomycetes and other microbes, and also means that the biodegradability (biodegradable rate and biodegradable degree) of the substance equals to a material naturally generated such as fallen leaves or a synthetic polymer generally recognized having the same biodegradability under the same environment. "Water dispersibility" has the same meaning as water solubility. It means a characteristic in which, while having no influence when used in a limited amount of moisture (menstrual blood), in a large amount of water or water current, the fabric is easily dispersed into small pieces at least to a degree where an ordinal toilet plumbing is not clogged. "Water solubility" is a characteristic in which, while having no influence when used in a limited amount of moisture (menstrual blood), the fabric is soluble in a large amount of water or water current.

### [Surface Side Sheet]

As the material for the surface side sheet for providing biodegradability, water dispersibility and water solubility, the spun lace nonwoven fabric can be used. It is preferable to use a wet spun lace nonwoven fabric with the fiber length of 1 to 15 mm. As other materials, so-called biodegradable resin such as polylactate and polybutylene succinate can be used. For example, a melt blown nonwoven fabric fabricated using polylactate as the raw material with a specific weight per unit area of 20 to 60 g / m² or a spun bond nonwoven fabric with the fineness of 1.0 to 3.0 dtex and a specific weight per unit area of 15 to 30 g / m² can be preferably used. Other material which can be used is a fiber obtained by providing tow with a specific weight per unit area of 10 to 80 g / m², which is a filament or continuous fiber of acetate, rayon, or synthetic fiber, and then disentangling the fibers.

### [Absorber]

As the material for the absorber for providing biodegradability, water dispersibility, and water solubility, the same material as that of the permeable surface side sheet can be used. It is also possible to use a single material selected from the group comprising an absorbing material such as algin soda, starch, carboxymethyl cellulose, a highly absorptive polymer grains and fibers. Or a sheet, which is obtained by mixing it with the same material as that of the above-described surface side sheet and forming it into a sheet, can be also used.

A specific example of the structure of the absorber, for example, is a material prepared by enclosing timber pulp by laminating it with a specific weight per unit area of 150 to 500 g / m² to tissue and prepare it to have a thickness of 2 to 10 mm by a pressing device. By mixing 5 to 30 g / m² of an absorbent such as starch with the above-mentioned absorber, it is possible to also improve the absorbance and menstrual blood holding capacity.

### [Back Side Sheet]

Examples of the impermeable material for the back side sheet which can provide biodegradability, water dispersibility, and water solubility at the same time are a polyvinyl alcohol (PVA) film, a film sheet in which repellent is applied by silicone resin or the like on the whole area or a part of one surface or both surfaces of the PVA film, a PVA film to which a silicone resin is mixed, a starch film, a film formed using so-called a biodegradable resin such as polylactate or polybuthylene succinate, and a laminated paper such as tissue. If necessary, coloring may be applied by mixing mineral pigment of 0.1 to 5 percent to these materials.

A specific example of the structure of the back side sheet is a laminated paper obtained by laminating a film formed of polylactate and tissue with a thickness of 10 to 20 µm and a specific weight per unit area of 20 to 50 g / m² by the laminating area ratio of 5 to 40 percent. Such laminated paper is preferable in respect that the impermeability can be maintained even when the pad is wet and there is no excessive burden imposed on septic tanks.

### [Mini Sheet Piece]

Examples of the material for the mini sheet piece for providing biodegradability, water dispersibility, and water solubility are a film formed of polylactate, polybutylene succinate, PVA and the like, or a laminated material obtained by laminating a film formed of these material and tissue.

### [Bonding Method]

As the applicable bonding method for providing biodegradability, water dispersibility, and water solubility, boding by polyvinyl alcohol with water solubility or water dilatation characteristic, heat sealing, hydrogen bonding may be used alone or combined to be used properly as a bonding method.

### [Functions]

The function regarding suppression of the number of live microorganism will be described by referring to sterilization as an example.

In the embodiment of the present invention, by sterilizing the interlabial pad packed in the individual wrapping container and the package through using gas and the like, it made possible to zero the number of the live microorganism in the interlabial pad immediately after the sterilization. Also, in the case where it is provided to have an over-wrapping structure in which contaminations hardly enter to the exterior container before opening, it becomes possible to suppress the number of the live microorganism in the interlabial pad to 100 or less in a period of 6 months after the sterilization. Furthermore, as for the exterior container, after opening, even being repeatedly opened and closed, the lid can cover the opening of the container so that the entering of contaminations can be suppressed as much as possible. Therefore, even after being opened by a user, the sanitary state of the outer environment of the sterilized interlabial pad enclosed in the individual wrapping container can be maintained.

Next, the function, which makes it possible to maintain the sanitary state of the interlabial pad while the user takes out the interlabial pad and wears it, will be described. The sterilized interlabial pad is enclosed in the individual wrapping container to which contaminations hardly enter so that the sanitary state is maintained. When the user opens the individual wrapping container, an interlabial pad whose surface side sheet hardly comes to be in contact with the finger appears from the opening of the individual wrapping container. Thereby, the user can take it out from the individual wrapping container and fix it between the labia without touching the surface side sheet of the interlabial pad by a finger and the like.

In addition, in the case where there is an opening for inserting a finger provided in the sterilized interlabial pad, it is possible to take out the pad while wearing the interlabial pad to the finger tip. Therefore, it can be precisely fixed between the labia as will be described later.

In the embodiment of the present invention, disclosed is an interlabial pad which is inserted between the female labia capable of directly adsorbing menstrual blood from the private part of the body. The sterilization is performed on the interlabial pad so that the number of live microorganism is suppressed to 100 or less even 6 months after the date of manufacture. By providing a using method in which, when the user of the interlabial pad fixes it between the labia, the contact with microorganism can be suppressed right before the skin-contact surface (surface member) of the interlabial pad touches (fix) the labia. That is, the wearing performance is provided in which a finger and the like, where indigenous microorganism thrive, do not touch the skin-contact surface (surface member) of the interlabial pad so that the state of equilibrium of indigenous microorganism near the labia of the user can be maintained when using the interlabial pad.

The functions other than the suppression of the number of live microorganism will be described below in order. The function of the surface side sheet is described. In the interlabial pad, the surface side sheet can permeate liquid so that the liquid discharged from the body can be immediately transmitted to the absorber.

Also, by fixing the interlabial pad to the body through adhesive on the surface side sheet, the gap generated between the interlabial pad and the body can be decreased without being influenced by the body movements. Thus, it becomes possible to completely shield the leak from the gap in the interlabial pad.

As the function of the absorber, it absorbs and holds the liquid permeated through the surface side sheet.

The functions of the back side sheet and the mini sheet piece will be described. The back side sheet has a sheet structure so that insert of the finger tip is not to be interrupted. When wearing, there may be cases where there is a decrease in the finger insert property caused by an increase in the friction drag between the inner side of the finger tip and the back side sheet due to the wet environment inside the finger tip to be inserted, or there is a fitting shift due to deterioration of the finger pulling property after wearing the interlabial pad. However, by reducing the surface contact area through performing a surface concave-convex processing on the back side sheet surface, changes in friction or sticking between the back side sheet and the finger tip, which is generated by the changes in the environment of the finger tip such as wet state or the like, can be avoided.

Unlike the product like napkins which are fixed to an underwear, when wearing the interlabial pad which is fixed to the body, it is hard to recognize the wearing position target (space between the labia or near the ostium of vagina). In the embodiment of the present invention, by having a structure in which a finger can be easily inserted, the interlabial pad can be temporarily held by the inserted finger, and the finger can be easily pulled out after wearing it between the labia, it becomes possible, when the wearer fixes the interlabial pad to the space between the labia, to have the inner side of the finger tip of the wearer with a keen sense be fixed facing the space between the labia of the wearer (or near the ostium of vagina). Therefore, even to the space between the labia which is hard to be viewed, the ostium of vagina can be detected by a natural act. As a result, not only simplifying the leading of the product to the right position but also a decrease in the leak due to wearing in a wrong position can be achieved.

Also, when inserting the finger tip to the interlabial pad, there may be a case where the inner side of the finger tip does not face the back side sheet but others. However, by opening (that is, the opening for inserting a finger is open) the periphery of the opening for inserting a finger of the interlabial pad in the projection from the front of the product, it becomes unnecessary for the wearer to insert the finger tip by opening the insertion opening through turning the finger or the like. Thereby, it becomes possible to have the finger tip with a keen sense readily facing the back side sheet (that is, the space between the labia or near the ostium of vagina) to be inserted.

Furthermore, by reducing the surface contact area of the mini sheet piece as in the same manner as the back side sheet, the influence by the changes in the wet environment of the finger tip can be avoided.

Also, by providing an elastic flexibility at least in the right and left hand side direction of the mini sheet piece, regardless of the finger tip size of the wearer, the interlabial pad can be temporarily fixed (held) to the finger tip and, without influencing the wearing state, the finger can be easily pulled out.

Also, the mini sheet piece from which the finger tip is pulled out is likely to be folded to the opposite side to the body by the wearing action due to its structural feature. Hence, when removing the used interlabial pad, the handling becomes easy by grabbing the mini sheet piece. Also, by providing the impermeability thereto, the mini sheet piece can be handled sanitarily without contaminating the finger.

The interlabial pad of the present invention may be used together with a sanitary napkin. There are some sanitary napkin users who use several pieces of napkins stacked together when there is a large quantity of menstrual blood, however, there causes problems that it feels uncomfortable such as stiffness and affects the external appearance. Also, the stacked sanitary napkins are put on one after another even near the ostium of vagina where there is no need for the stacked napkins, which causes rash and stuffiness. However, according to the embodiment, the sanitary products are stacked only in the area between the labia. Therefore, there is little influence on the wear feeling and the external appearance. Also, rash and stuffiness in the breech and its periphery can be decreased.

Furthermore, when changing the pad, it becomes possible to change only the interlabial pad according to the present invention without changing the sanitary napkin. Therefore, there is no need for the wearer to carry around the sanitary napkins which are large enough to be noticed.

In the present invention, the interlabial pad is wrapped anisotropic to the wrapping container. Therefore, the opening direction and the finger insert direction can be made in the same direction for the wearer so that the wearer can insert the finger more easily.

The interlabial pad 1 according to the embodiment can be used together with an ordinal sanitary napkin. As the wearing method, the interlabial pad is fixed between the labia and the sanitary napkin is fixed to the underwear. Thereby, even on a day with a large quantity of menstrual blood, the interlabial pad of the present invention can be effectively used.

### [Details of Sample Used in Test]

As for the material structure, a spun lace nonwoven fabric was used for the surface side sheet. The blending proportion of the nonwoven fabric was rayon / polyester = 85 / 15. The fineness of the rayon was 1.7 dtex and that of the polyester was 2.8 dtex. A specific weight per unit area was 40 g / m².

A fiber aggregate was used for the absorber. The blending proportion was rayon / natural cotton = 75 / 25. The fineness of the rayon was 3.3 dtex and a specific weight per unit area was 350 g / m². For the back side sheet, a polyethylene film with a film thickness of 20 µm was used. For the mini sheet piece, a polypropylene SMS nonwovwen fabric with a specific weight per unit area of 18 g / m² was used.

The size of the elliptic interlabial pad was 100 mm in the vertical axis direction and 65 mm in the horizontal axis direction. The product weight was 3 g.

For the individual wrapping, a compound body of spun bond nonwoven fabric and polyethylene film was used. The spun bond nonwoven fabric was polyethylene / polypropylene (sheath / core) with a specific weight per unit area of 22 g / m². The film thickness of the polyethylene film was 10µm, which was fabricated by a press-laminate processing.

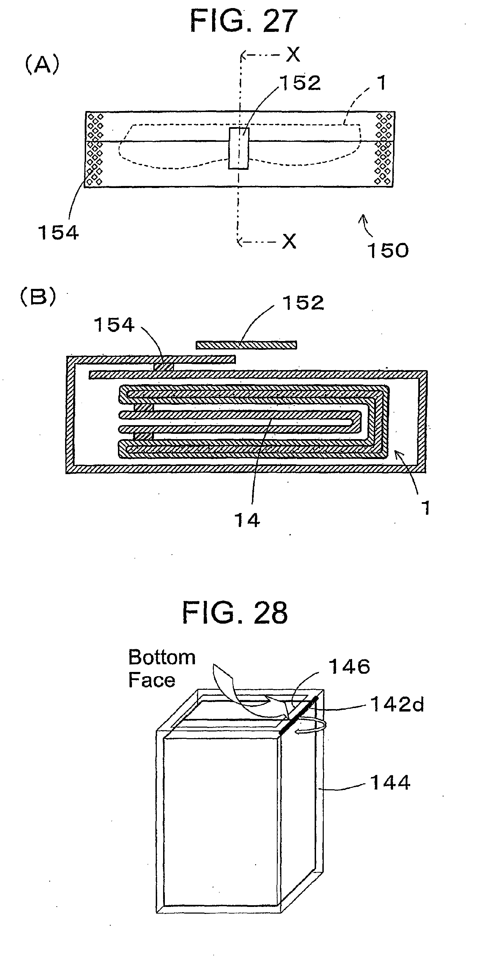

As shown in Fig. 27, the surface side sheet was folded in two towards the outside along the vertical axis direction, and the interlabial pad was enclosed in such manner that the opening of the individual wrapping container and the opening for inserting a finger of the pad were to be in the same direction. The edge of the individual wrapping container was bonded by lattice embossing and, to provide breathing characteristic, dot embossing was employed.

The interlabial pad 1 was folded in two along the longitudinal direction with the mini sheet piece 14 being inside. The interlabial pad was enclosed so that, when pulling a tub tape 152, the opening of the individual wrapping container and the opening for inserting a finger of the pad were to be in the same direction. The numeral 154 was an adhesive.

For the package, as shown in Fig. 23, a cardboard was used and, as shown in Fig. 24, polypropylene shrink film was used for the over-wrapping film. The film thickness of the over-wrapping film was 25 µm. The gaps in the container bottom face of the exterior container and the gaps of the lid were over-wrapped by the polypropylene shrink film.

As shown in Fig. 28, the over-wrapping film has a gap 146 and a gap 142d in the bottom face so that ethylene oxide gas can enter inside a package 140.

The size of the interlabial pad enclosed in the individual wrapping container was 130 mm in the longitudinal direction and 40 mm in the lateral direction.

The size of the exterior container was 105 cm in width, 105 mm in depth, and 130 mm in height. Thirty pieces of the interlabial pads enclosed in the individual wrapping container were placed in the exterior container.

The sterilization condition using ethylene oxide was as follows. The gas used was a mixed gas with 20 percent of ethylene oxide and 80 percent of carbon dioxide. The inner pressure of the exterior container to which the interlabial pad was enclosed was prepared to be -91.2 kPa. Then, the above-described gas was introduced to make the inner pressure to be 50 kPa and inside the container was heated to 50 °C. Inside the container was kept at 50 °C for 4 hours to be pasteurized. Subsequently, the ethylene oxide gas was removed from the interlabial pad. When removing, the inner pressure was decreased to be -91.2 kPa or less and then air was introduced to restore the pressure to the atmospheric pressure. This operation was performed once.

Sterility Test was carried out as follows. The medium used is based on Japanese version of the Japanese Pharmacopoeia, Fourteenth Edition, General Tests, No. 59 Sterility Test (No. 54 in English version). The testing method employs a direct method and the basic operation follows the above-described testing method.

For the preparation of test solution, the prepared medium is inserted to cultural test tubes of φ 30 mm x 120 mm by 45 ml each and caps provided exclusively were applied. The tubes were autoclaved at 121 °C for 20 minutes. After cooling, one piece of the interlabial pad was inserted (tested in two tubes per test sample).

Subsequently, according to Pharmacopoeia, the tubes were left in an incubator for 14 days for cultivation.

Cultivation was performed on a thioglycolate acid medium at 30 to 35 °C and on SCD medium at 20 to 25 °C. Observation and judgment were performed according to Pharmacopoeia.

A microbe limit test was carried out as follows. The medium used was based on Japanese version of the Japanese Pharmacopoeia, Fourteenth Edition, General Tests, No. 50 Microbial Limit Test (No. 35 in English version).

Soybean casein digest (SCD) agar culture medium and antibiotic-added Sabouraud dextrose agar culture medium were prepared.

The testing method was agar flat-plate mixing method (according to Japanese version of the Japanese Pharmacopoeia, Fourteenth Edition, General Tests No. 50 Microbial Limit Test (No. 35 in English version)). 100 ml of pepton salt buffer solution (according to Japanese version of the Japanese Pharmacopoeia, Fourteenth Edition, General Tests, No. 50 Microbial Limit Test (No. 35 in English version)) was prepared. 10 g of the interlabial pad (prepared as insoluble solid agent) was put in a plastic bag and was stomached for 1 minute by a stomacher to obtain a test undiluted solution. The sample was preserved under a specific environment with a temperature at 30 °C and the humidity of 80 percent.

The results are shown below:

| DATA of Sterilization to 6months later | | | | | | | | |
|---|---|---|---|---|---|---|---|---|
| No. of Live microorganism / p | | | | | | | | |
| | After Manufacture | Right After Sterilization | 1 mth | 2mth | 3mth | 4mth | 5mth | 6mth |
| Sterilization-Done | 210 / p | 0 | 0 | 0 | 0 | 0 | 0 | 0 |
| | | | | | | | | |
| No. of Bacteria | 210 / p | 0 | 0 | 0 | 0 | 0 | 0 | 0 |
| No. of Fungi | 0 / p | 0 | 0 | 0 | 0 | 0 | 0 | 0 |
| | | | | | | | | |
| No Sterilization(blank) | 210 / p | ― | 210 | 210 | 200 | 190 | 180 | 180 |
| No. of Bacteria | 210 / p | ― | 210 | 210 | 200 | 190 | 180 | 180 |
| No. of Fungi | 0 / p | 0 | 0 | 0 | 0 | 0 | 0 | 0 |

### Industrial Applicability

As described, in the present invention, the number of live microorganism is suppressed to be 100 or less so that the state of equilibrium of indigenous microorganism near the labia can be maintained. Also, when a user wears the interlabial pad, a finger or the like of the user does not touch the skin-contact surface of the interlabial pad. Therefore, the interlabial pad can be attached between the labia while keeping the sanitary state.

## Claims

1. An individual wrapping body comprising:
an interlabial pad with a size capable of being smoothly inserted between female labia; and
an individual wrapping container for covering and enclosing the whole portion of said interlabial pad,
wherein a processing for suppressing the number of live microorganism is applied to said interlabial pad during manufacturing and / or after manufacturing, and the number of live microorganism is suppressed to equal to 100 or less even after a period of six months from the manufacture.

2. An individual wrapping body as claimed in claim 1, wherein said processing is a disinfection processing.

3. An individual wrapping body as claimed in claim 2, wherein, by said disinfection processing, said interlabial pad is sterilized immediately after manufacturing.

4. An individual wrapping body as claimed in any one of claims 1 to 3, wherein said interlabial pad comprises:
a permeable surface side sheet facing a body side;
a back side sheet facing a garment side, the back side sheet being bonded to said surface side sheet; and
an absorber included between said surface side sheet and said back side sheet.

5. An individual wrapping body as claimed in any one of claims 1 to 4, further comprising a mini sheet piece on the garment side surface of said back side sheet,
said mini sheet piece having one or more bonded areas in each side area in the longitudinal direction of said back side sheet; and
an unbonded area in the lateral direction of said back side sheet; said unbonded area forming an opening for inserting a finger between said mini sheet piece and said back side sheet.

6. An individual wrapping body as claimed in claim 5, wherein said interlabial pad is folded and enclosed such that said opening for inserting a finger opens.

7. An individual wrapping body as claimed in claim 5 or 6, wherein an interlabial pad is positioned such that said opening for inserting a finger is positioned to face the opening of said individual wrapping body.

8. An exterior container, comprising a package for packaging two or more said individual wrapping bodies as claimed in any one of claims 1 to 7, wherein said package comprises:
a main body of a container and
a lid capable of opening and closing for covering an opening part of said main body of the container.

9. An exterior container as claimed in claim 8, wherein said package includes an over-wrapping for covering at least said main body of the container.

10. An exterior container as claimed in claim 8 or 9, wherein said main body of the container of said package is bonded with at least a part of said lid.

11. An exterior container as claimed in claim 8 or 9, wherein said main body of the container of said package and said lid are continuously formed; and said package is formed by bonding said lid towards said main body of the container.
